# EUROPEAN PATENT APPLICATION

(11) **EP 4 748 929 A2**
(43) Date of publication of application: **27.05.2026**
(21) Application number: 26163068.5
(22) Date of filing: 02.03.2022
(51) Int. Cl.: C12N 9/16

(54) **NOVEL CRISPR-CAS NUCLEASES FROM METAGENOMES**

(30) Priority: 02.03.2021 EP 21000063
(62) Divisional of application: 22707784.9
(71) Applicant: BRAIN Biotech AG, 64673 Zwingenberg (DE)
(72) Inventor: SCHOLZ, Paul, 50679 Köln (DE); ZUREK, Christian, 64653 Lorsch (DE); BACK, Robin, 64625 Bensheim (DE)
(74) Representative: Weickmann & Weickmann PartmbB

(57) **Abstract**

The present invention relates to a nucleic acid molecule encoding an RNA-guided DNA endonuclease, which is (a) a nucleic acid molecule encoding the RNA-guided DNA endonuclease comprising or consisting of the amino acid sequence of any one of SEQ ID NOs 9, 1 to 5, 7, 8 and 10 to 15; (b) a nucleic acid molecule comprising or consisting of the nucleotide sequence of any one of SEQ ID NOs 24, 16 to 20, 22, 23 and 25 to 30; (c) a nucleic acid molecule encoding a RNA-guided DNA endonuclease the amino acid sequence of which is at least 70% identical to the amino acid sequence of (a), preferably at least 80 % identical, more preferably at least 90% identical, and most preferred at least 95% identical; (d) a nucleic acid molecule comprising or consisting of a nucleotide sequence which is at least 70% identical to the nucleotide sequence of (b), preferably at least 80 % identical, more preferably at least 90% identical, and most preferred at least 95% identical; (e) a nucleic acid molecule which is degenerate with respect to the nucleic acid molecule of (d); or (f) a nucleic acid molecule corresponding to the nucleic acid molecule of any one of (a) to (d) wherein T is replaced by U.

## Description

The present invention relates to a nucleic acid molecule encoding an RNA-guided DNA endonuclease, which is (a) a nucleic acid molecule encoding the RNA-guided DNA endonuclease comprising or consisting of the amino acid sequence of any one of SEQ ID NOs 9, 1 to 5, 7, 8 and 10 to 15; (b) a nucleic acid molecule comprising or consisting of the nucleotide sequence of any one of SEQ ID NOs 24, 16 to 20, 22, 23 and 25 to 30; (c) a nucleic acid molecule encoding a RNA-guided DNA endonuclease the amino acid sequence of which is at least 70% identical to the amino acid sequence of (a), preferably at least 80 % identical, more preferably at least 90% identical, and most preferred at least 95% identical; (d) a nucleic acid molecule comprising or consisting of a nucleotide sequence which is at least 70% identical to the nucleotide sequence of (b), preferably at least 80 % identical, more preferably at least 90% identical, and most preferred at least 95% identical; (e) a nucleic acid molecule which is degenerate with respect to the nucleic acid molecule of (d); or (f) a nucleic acid molecule corresponding to the nucleic acid molecule of any one of (a) to (d) wherein T is replaced by U.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

CRISPR-Cas systems are widespread adaptive immunity systems of prokaryotes against invading foreign nucleic acids. So far, more than 30 different CRISPR-Cas systems have been identified that differ in their loci architecture, number, and identity of their genes encoding for the Cas (CRISPR-associated) proteins.

The typical signature of the CRISPR systems in prokaryotic genomes is the presence of short (30-45 bp) repetitive sequences (repeats) that are intervened by variable sequences (spacers) of similar lengths. The Cas proteins are located either upstream or downstream of the repeat-spacer cluster. According to their gene composition and mechanistic differences, the subtypes are classified into two CRISPR classes (Class 1 and 2). One of their major differences is that Class 1 CRISPR systems need a complex of multiple Cas proteins to degrade DNA, whereas Class 2 Cas proteins are single, large multidomain nucleases. The sequence-specificity of the Class 2 Cas proteins can simply be modified by synthetic CRISPR RNAs (crRNAs) in order to introduce targeted double-stranded DNA breaks. Most prominent members of such Class 2 Cas proteins are Cas9, Cpf1 (Cas12a) and Cms1, which are harnessed for genome-editing and successfully applied in many eukaryotic organisms including fungi, plants and mammalian cells. Whereas Cas9 and its orthologs are Class2 type II CRISPR nucleases, Cpf1 (WO2016/205711 BROAD Inst.; WO2017/141173 Benson Hill) and Cms1 (WO2019/030695 Benson Hill) belong to Class 2 type V nucleases. Cms1 and Cpf1 CRISPR nucleases are a class of CRISPR nucleases that have certain desirable properties compared to other CRISPR nucleases such as type II nucleases. For instance, in contrast to Cas9 nucleases Cms1 and Cpf1 do not require a trans-activating crRNA (tracrRNA), which is partially complementary to the precursor crRNA (pre-crRNA) (Deltcheva et al. (2011), Nature, 471(7340):602-607). Base-pairing of tracrRNA and pre-crRNA forms a Cas9-bound RNA:RNA duplex, which gets processed by RNase III and other unidentified nucleases. This mature tracrRNA:crRNA duplex mediates the target DNA recognition and cleavage by Cas9. In contrast, type V nucleases can process the pre-crRNA without the need for tracrRNA or cellular nucleases (like RNase III), which significantly simplifies the application of type V nucleases for (multiplex) genome editing.

Several new Class 2 proteins, like C2c1 (Cas12b), C2c2 (Cas13a) and C2c3 (Cas12c) have been identified in the genomes of cultivated bacteria or public available metagenomics datasets, e.g. gut metagenome (Shmakov et al. (2015), Mol Cell,60(3):385-97). According to the recent classification of CRISPR-Cas systems, Class 2 comprises 3 types and 17 subtypes (Makarova et al. (2020), Nat Rev Microbiol, 18(2):67-83).

Moreover, in a recent publication two new Class 2 proteins were discovered (CasX (Cas12a) and CasY (Cas12d)) in uncultivated prokaryotes by metagenome sequencing (Burstein et al. (2017), Nature, 542:237-241), indicating the presence of untapped Cas proteins from organisms which are not cultivated and/or identified yet.

A promising approach for the discovery of novel Class 2 Cas proteins is therefore to access the metagenomics resource by next generation sequencing selected environmental DNA (e.g. 1 cm³ forest soil contains ~ 2.5 x 10¹⁰ bp DNA or ~ 20 million genes) and computational identification of CRISPR-Cas systems (for example, Lei and Sun (2016), Bioinformatics, 32(17):i520-i528).

As discussed, the known CRISPR-Cas systems display certain differences as regards their mode of action. These molecular differences not only enlarge the possibilities of using the CRISPR-Cas system for genome editing in a broad range of different genetic backgrounds but also to circumvent issues of particular Cas nucleases when applied in certain organisms, e.g. pre-existing immune response to Cas9 in humans (Charlesworth et al. (2019), Nat Med, 25(2):249-254). Therefore, the identification of Cas nucleases from bacterial species with less direct contact to higher eukaryotes is of particular importance. It can be assumed that a number of yet undiscovered CRISPR-Cas systems exist with yet unknown characteristics, in particular in metagenome resources. Hence, although already several different CRISPR-Cas systems are known from the prior art there is an ongoing need to identify further CRISPR-Cas systems and in particular the RNA-guided DNA endonucleases of these systems. This need is addressed by the present invention

Accordingly, the present invention relates in first aspect to a nucleic acid molecule encoding an RNA-guided DNA endonuclease, which is (a) a nucleic acid molecule encoding the RNA-guided DNA endonuclease comprising or consisting of the amino acid sequence of any one of SEQ ID NOs 1 - 15, preferably of SEQ ID NOs 9, 1 to 5, 7, 8 and 10 to 15 or of SEQ ID NO: 6; (b) a nucleic acid molecule comprising or consisting of the nucleotide sequence of any one of SEQ ID NOs 16 - 30, preferably of SEQ ID NOs 9,1 to 5, 7, 8 and 10 to 15 or of SEQ ID NO: 22; (c) a nucleic acid molecule encoding a RNA-guided DNA endonuclease the amino acid sequence of which is at least 70% identical to the amino acid sequence of (a), preferably at least 80% identical, more preferably at least 90% identical, and most preferred at least 95% identical; (d) a nucleic acid molecule comprising or consisting of a nucleotide sequence which is at least 70% identical to the nucleotide sequence of (b), preferably at least 80% identical, more preferably at least 90% identical, and most preferred at least 95% identical; (e) a nucleic acid molecule which is degenerate with respect to the nucleic acid molecule of (d); or (f) a nucleic acid molecule corresponding to the nucleic acid molecule of any one of (a) to (d) wherein T is replaced by U.

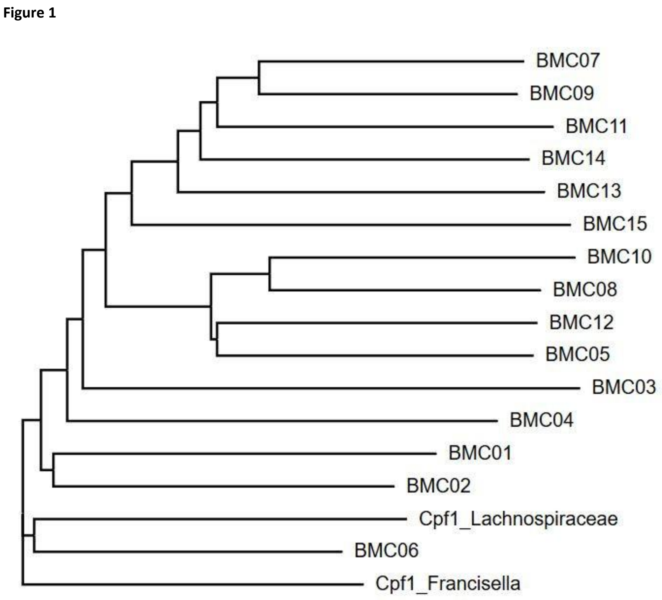

SEQ ID NOs 1 - 15 are the amino acid sequences of the novel CRISPR-Cas endonucleases BMC01 - BMC15, respectively, wherein BMC is an abbreviation of BRAIN Metagenome Cas. The novel CRISPR-Cas endonucleases BMC01 - BMC15 are encoded by the nucleotide sequences of SEQ ID NOs 16 - 30, respectively. An unrooted tree of the amino acid sequences of BMC01 - BMC15 is shown in Figure 1 of the application. It can be seen that BMC01 to BMC05 and BMC07 to BMC15 are closer related to each other than to any prior art CRISPR-Cas endonuclease the application is aware of and are closer related to each other than to BMC06. For this reason SEQ ID NOs 1 - 15 are preferably SEQ ID NOs 9, 1 to 5, 7, 8 and 10 to 15 or is SEQ ID NO: 6, and SEQ ID NOs 16 to 30 are preferably SEQ ID NOs 24, 16 to 20, 22, 23 and 25 to 30 or is SEQ ID NO: 22. Among SEQ ID NOs 9, 1 to 5, 7, 8 and 10 to 15 SEQ ID NO: 9 is mentioned first because the corresponding novel CRISPR-Cas endonuclease BMC09 has been most extensively tested in the appended examples. For this reason the amino acid sequence of SEQ ID NO: 9 as well as the corresponding nucleotide sequence of SEQ ID NO: 24 are most preferred.

In accordance with the present invention the term "nucleic acid molecule" defines a linear molecular chain of nucleotides. The nucleic acid molecules according to the present invention consist of at least 2327 nucleotides. The group of molecules designated herein as "nucleic acid molecules" also comprises complete genes. The term "nucleic acid molecule" is interchangeably used herein with the term "polynucleotide".

The term "nucleic acid molecule" in accordance with the present invention includes DNA, such as cDNA or double or single stranded genomic DNA and RNA. In this regard, "DNA" (deoxyribonucleic acid) means any chain or sequence of the chemical building blocks adenine (A), guanine (G), cytosine (C) and thymine (T), called nucleotide bases, that are linked together on a deoxyribose sugar backbone. DNA can have one strand of nucleotide bases, or two complimentary strands which may form a double helix structure. "RNA" (ribonucleic acid) means any chain or sequence of the chemical building blocks adenine (A), guanine (G), cytosine (C) and uracil (U), called nucleotide bases, that are linked together on a ribose sugar backbone. RNA typically has one strand of nucleotide bases. Included are also single-and double-stranded hybrid molecules, i.e., DNA-DNA, DNA-RNA and RNA-RNA. The nucleic acid molecule may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Polynucleotides may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. The polynucleotides may be derivatized by formation of a methyl or ethyl phosphotriester or an alkyl phosphorarnidate linkage. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. Also included are nucleic acids containing modified bases, for example thio-uracil, thio-guanine and fluoro-uracil. A nucleic acid molecule typically carries genetic information, including the information used by cellular machinery to make proteins and/or polypeptides. The nucleic acid molecule of the invention may additionally comprise promoters, enhancers, response elements, signal sequences, polyadenylation sequences, introns, 5'-and 3'- non-coding regions, and the like.

The term "polypeptide" as used herein interchangeably with the term "protein" describes linear molecular chains of amino acids, including single chain proteins or their fragments. The polypeptides / proteins according to the present invention contain at least 775 amino acids. Polypeptides may further form oligomers consisting of at least two identical or different molecules. The corresponding higher order structures of such multimers are, correspondingly, termed homo- or heterodimers, homo- or heterotrimers etc.. The polypeptides of the invention may form heteromultimers or homomultimers, such as heterodimers or homodimers. Furthermore, peptidomimetics of such proteins/polypeptides where amino acid(s) and/or peptide bond(s) have been replaced by functional analogues are also encompassed by the invention. Such functional analogues include all known amino acids other than the 20 gene-encoded amino acids, such as selenocysteine. The terms "polypeptide" and "protein" also refer to naturally modified polypeptides and proteins where the modification is affected e.g. by glycosylation, acetylation, phosphorylation, ubiquitinylation and similar modifications which are well known in the art.

The term "RNA-guided DNA endonuclease" or "CRISPR(-Cas) endonuclease" describes an enzyme having the capability of cleaving the phosphodiester bond within a deoxyribonucleotide (DNA) strand thereby producing a double-strand break (DSB). BMC01 - 15 are classified as novel type V class 2 CRISPR nucleases which are known to introduce a staggered cut with a 5' overhang. Hence, an RNA-guided DNA endonuclease comprises an endonuclease domain, in particular a RuvC domain. The RuvC domains of BMC01 - 15 is believed to comprise three split RuvC motifs. A RNA-guided DNA endonuclease also comprises a domain being capable of binding to a crRNA, also known as guide RNA (gRNA; also being designated DNA-targeting RNA herein).

The cleavage site of the RNA-guided DNA endonuclease is guided by a guide RNA. The gRNA confers the target sequence specificity to the RNA-guided DNA endonuclease. Such gRNAs are non-coding short RNA sequences which binds to the complementary target DNA sequences. The gRNA first binds to the RNA-guided DNA endonuclease by a binding domain that can interact with the RNA-guided DNA endonuclease. The binding domain that can interact with the RNA-guided DNA endonuclease typically comprises a region with a stem-loop structure. This stem-loop preferably comprises the sequence NATTTCTACTNTTGTAGAT (SEQ ID NO: 31), wherein N denotes that any base may be present at this location. The stem-loop most preferably comprises the stem loop direct repeat sequence of BMC01-BMC15 (SEQ ID NO: 32), respectively, but in the form of RNA (i.e. wherein T is replaced by U). The gRNA sequence guides the complex (known as CRISPR ribonucleoprotein (RNP) complex of the gRNA and the RNA-guided DNA endonuclease) via pairing to a specific location on a DNA strand, where the RNA-guided DNA endonuclease performs its endonuclease activity by cutting the DNA strand at the target site. The genomic target site of the gRNA can be any about 20 (typically 17 to 26) nucleotide DNA sequence, provided it meets two conditions: (i) The sequence is unique compared to the rest of the genome, and (ii) the target is present immediately adjacent to a Protospacer Adjacent Motif (PAM).

The cleavage site of the RNA-guided DNA endonuclease is, thus, furthermore defined by a PAM. The PAM is a short DNA sequence (usually 2-6 base pairs in length) that follows the DNA region targeted for cleavage by the CRISPR system. The exact sequence depends on which CRISPR endonuclease is used. CRISPR endonucleases and their respective PAM sequences are known in the art (see https://www.addgene.org/crispr/guide/#pam-table). For instance, the PAM being recognized by the first identified RNA-guided DNA endonuclease Cas9 is 5'-NGG-3' (where "N" can be any nucleotide base). The PAM is required for a RNA-guided DNA endonuclease to cut. In Cas9 it is found about 2-6 nucleotides downstream of the DNA sequence targeted by the guide RNA and 3-6 nucleotides downstream from the cut site. In type V systems (including BMC01 - BMC15) the PAM is located upstream of both, the target sequence and the cleavage site. The complex of the RNA-guided DNA endonuclease and the guide RNA comprises a so-called PAM interacting domain (Andres et al. (2014), Nature, 513(7519):569-573). Hence, the genomic locations that can be targeted for editing by an RNA-guided DNA endonuclease are limited by the presence and locations of the nuclease-specific PAM sequence. As BMC01 - BMC15 belong to the group of Type V Class 2 CRISPR nucleases a T rich PAM site is predicted (TTTN, wherein N can be any nucleotide of A, T, C and G).

The term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 70% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Amino acid sequence as well as nucleotide sequence analysis and alignments in connection with the present invention are preferably carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402). The skilled person is aware of additional suitable programs to align nucleic acid sequences.

As defined herein above, amino acid sequence and nucleotide sequence identities of at least 70%, at least 80%, at least 90% and at least 95% are envisaged by the invention. Furthermore, are envisaged with increasing preference amino acid sequence identities of at least 99.5 %, at least 99.8 %, and 99.9% identity by the invention.

With respect to these amino acid sequences and the amino acid sequences being encoded by these nucleotide sequences it is preferred that they maintain or essentially maintain the RNA-guided DNA endonuclease activity of SEQ ID NOs 1 - 16 of the invention. Hence, what is maintained or essentially maintained is the capability to bind to the gRNA to form a complex being capable of binding to the DNA target site of interest, where the endonuclease activity induces a DSB.

The term "degenerate" designates the degeneracy of the genetic code. As is well known, the codons encoding one amino acid may differ in any of their three positions; however, more often than not, this difference is in the second or third position. For instance, the amino acid glutamic acid is specified by GAA and GAG codons (difference in the third position); the amino acid leucine is specified by UUA, UUG, CUU, CUC, CUA, CUG codons (difference in the first or third position); and the amino acid serine is specified by UCA, UCG, UCC, UCU, AGU, AGC (difference in the first, second, or third position).

Just as the type V CRISPR endonucleases Cms1 and Cpf1, BMC01 - BMC15 do not require a trans-activating crRNA (tracrRNA). Moreover, the BMC01 - BMC15 containing CRISPR systems identified in the present application contain CRISPR repeat sequences with an RNA stem loop at 3'-ends of each repeat that is conserved in crRNAs of Cpf1 and Cms1 protein families and the "nearest neighbour" of BMC01 - BMC15 among the known CRISPR-Cas endonucleases are members of the Cpf1 polypeptides. For this reason BMC01 - BMC15 can be classified as novel Class 2 type V nucleases with overall no significant sequence identity to the known collection of Class 1 and Class 2 CRISPR-Cas endonucleases and with overall low sequence identity to individual Cpf1-type endonucleases.

Since BMC01 - BMC15 are novel CRISPR-Cas endonucleases which are significantly distinct from the known collection of CRISPR-Cas endonucleases, BMC01 - BMC15 expand the known collection of CRISPR-Cas endonucleases applicable for genome editing, gene regulation and nucleic acid enrichment/purification in different biotechnological and pharmaceutical sectors thereby advantageously expanding the options for molecular genome editing.

BMC01 - BMC15 are significantly distinct from the known collection of known CRISPR-Cas endonucleases in at least two aspects.

First, they are structurally distinct. For instance, SEQ ID NO: 20 in WO 2021/154866 only shares a sequence similarity of 57.1% with CMS09 (SEQ ID NO: 9) and a sequence identity of 55.26% with the hypothetical protein US54 C0016G0015 from a *Candidatus Roizmanbacteria* bacterium. Also the BMC endonucleases are structurally significantly distinct from known CRISPR-Cas endonucleases. The BMC endonucleases were found by the innovative screening approach as illustrated in Example 1, wherein metagenomics resources in terabase scale were accessed by Next Generation Sequencing and Candidata Phyla Radiation (CPR) species containing a nearly untouched space of novel sequences were enriched. It was surprising that despite the structurally significantly distinct sequences as compared to the known CRISPR-Cas endonucleases, BMC01 - BMC15 still work very well for genome editing, which is demonstrated by Examples 3 to 5.

Second, BMC01 - BMC15 were isolated from environmental samples, in particular from aquatic, sediment and soil habitats. On the other hand, most known CRISPR-Cas endonucleases were isolated from pathogenic bacteria. For instance, Cas9 was found in *Streptococcus pyogenes* M1, Cas12a (Cpf1) from *Prevotella* and *Francisella* 1, and Cas13a (C2c2) from *Leptotrichia shahii.* For this reason BMC01 - BMC15 will be less immunogenic than most known CRISPR-Cas endonucleases when applied for genome editing in mammals, preferably in humans in particular for therapeutic applications.

In accordance with a preferred embodiment of the first aspect of the invention the nucleic acid molecule is operably linked to a promoter that is native or heterologous to the nucleic acid molecule.

A promoter is a region of DNA that leads to initiation of transcription of a particular gene. Promoters are generally located near the transcription start sites of genes, upstream on the DNA (towards the 5' region of the sense strand). Promoters are typically 100-1000 base pairs long. For transcription to take place, the enzyme that synthesizes RNA, known as RNA polymerase, must attach to the DNA near a gene. Promoters contain specific DNA sequences such as response elements that provide a secure initial binding site for RNA polymerase and for proteins called transcription factors that recruit RNA polymerase. Hence, the binding of the RNA polymerase and transcription factors to the promoter site ensures the transcription of the gene.

In this connection the term "operably linked" defines that the promoter is linked to the gene of the same DNA strand, such that upon binding of the RNA polymerase and transcription factors the transcription of the gene is initiated. Generally each gene is operably linked in its natural environment of the genome of a living organism to a promoter. This promoter is designated "natural promoter" or "wild-type promoter" herein. A heterologous promoter is distinct from the natural promoter or wild-type promoter. Hence, a nucleic acid molecule which is operably linked to a promoter that is heterologous to the nucleic acid molecule does not occur in nature.

Heterologous promoters that can be used to express a desired gene are known in the art and can, for example, be obtained from the EPD (eukaryotic promoter database) or EDPnew (https://epd.epfl.ch//index.php). In this database eukaryotic promoters including animal, plant and yeast promoters can be found.

The promoter can, for example, be a constitutively active, inducible, tissue-specific, or developmental stage-specific promoter. By using such promoter the desired timing and site of expression can be regulated.

The *AOX1* or *GAL1* promoter in yeast or the CMV- (Cytomegalovirus), SV40-, RSV-promoter (Rous sarcoma virus), chicken beta-actin promoters, CAG-promoter (a combination of chicken beta-actin promoter and cytomegalovirus immediate-early enhancer), the gai10 promoter, human elongation factor 1α-promoter, CaM-kinase promoter, and the Autographa californica multiple nuclear polyhedrosis virus (AcMNPV) polyhedral promoter are examples of constitutively active promoters.

Examples of inducible promoters are the Adhl promoter which is inducible by hypoxia or cold stress, the Hsp70 promoter which is inducible by heat stress, the PPDK promoter and the pepcarboxylase promoter which are both inducible by light. Also useful are promoters which are chemically inducible, such as the In2-2 promoter which is safener induced (US 5,364,780), the ERE promoter which is estrogen induced, and the Axigl promoter which is auxin induced and tapetum specific but also active in callus (WO03060123 A1).

A tissue-specific promoter is a promoter that initiates transcription only in certain tissues. A developmental stage-specific promoter is a promoter that initiates transcription only in a certain developmental stage.

In accordance with a further preferred embodiment of the first aspect of the invention the nucleic acid molecule is linked to a nucleic acid sequence encoding a nuclear localization signal (NLS).

Further details on NLS will be provided herein below.

In accordance with another preferred embodiment of the first aspect of the invention said nucleic acid molecule is codon-optimized for expression in a eukaryotic cell, preferably a plant cell or an animal cell.

As discussed, BMC01 - BMC15 were isolated form bacterial metagenome samples. Hence, if BMC01 - BMC15 are to be expressed in eukaryotic cells they are expressed in a heterologous host cell or organism.

The gene encoding BMC01 - BMC15 polypeptide, respectively, can be codon-optimized for expression in the target cell, and can optionally include a sequence encoding an NLS and/or a peptide tag, such a purification tag. Further details on the tag will be provided herein below.

Codon optimization is a process used to improve gene expression and increase the translational efficiency of a gene of interest by accommodating codon bias of the host cell. A "codon-optimized gene" is therefore a gene having its frequency of codon usage designed to mimic the frequency of preferred codon usage of the host cell. Nucleic acid molecules can be codon optimized, either wholly or in part. Because any one amino acid (except for methionine and tryptophan) is encoded by a number of codons, the sequence of the nucleic acid molecule may be changed without changing the encoded amino acid. Codon optimization is when one or more codons are altered at the nucleic acid level such that the amino acids are not changed but expression in a particular host organism is increased. Those having ordinary skill in the art will recognize that codon tables and other references providing preference information for a wide range of organisms are available in the art (see, e.g., Zhang et al. (1991) Gene 105:61-72; Murray et al. (1989) Nucl. Acids Res. 17:477-508). Methodology for optimizing a nucleotide sequence for expression is provided, for example, in U.S. Pat. No. 6,015,891. Programs for codon optimization are available in the art (e.g., OPTIMIZER at genomes.urv.es/OPTIMIZER; OptimumGene.TM. from GenScript at:
www.genscript.com/codon_opt.html).

The eukaryotic cell is preferably a Chinese hamster ovary (CHO) cell and accordingly the codon-optimization is preferably an optimization for the expression in CHO cells. CHO cells are of particular commercial interest since they are the most commonly used mammalian hosts for the industrial production of recombinant protein therapeutics.

Further details on suitable eukaryotic cells, including plant and animal cells will be provided herein below.

The present invention relates in a second aspect to a vector encoding the nucleic acid molecule of the first aspect.

The definitions and preferred embodiments as described herein above apply *mutatis mutandis* to the second aspect, if applicable.

A vector according to this invention is generally and preferably capable of directing the replication, and/or the expression of the nucleic acid molecule of the invention and/or the expression of the polypeptide encoded thereby.

Preferably, the vector is a plasmid, cosmid, virus, bacteriophage or another vector used conventionally e.g. in genetic engineering.

Exemplary plasmids and vectors are listed, for example, in Studier and coworkers (Studier, W.F.; Rosenberg A.H.; Dunn J.J.; Dubendroff J.W., 1990, Use of the T7 RNA polymerase to direct expression of cloned genes, Methods Enzymol. 185, 61-89) or the brochures supplied by the companies Novagen, Promega, New England Biolabs, Clontech and Gibco BRL. Other preferred plasmids and vectors can be found in: Glover, D.M., 1985, DNA cloning: a practical approach, Vol. I-III, IRL Press Ltd., Oxford; Rodriguez, R.L. and Denhardt, D.T. (eds), 1988, Vectors: a survey of molecular cloning vectors and their uses, 179-204, Butterworth, Stoneham; Goedeel, D.V., 1990, Systems for heterologous gene expression, Methods Enzymol. 185, 3-7; Sambrook, J.; Russell, D. W., 2001, Molecular cloning: a laboratory manual, 3rd ed., Cold Spring Harbor Laboratory Press, New York.

Particularly preferred vectors are vectors that can be used for CRISPR genome editing, in particular vectors only expressing the nucleic acid molecule of the invention encoding the RNA-guided DNA endonuclease or vectors expressing both, the nucleic acid molecule of the invention encoding the RNA-guided DNA endonuclease and the guide RNA (so called "all-in one vectors"). In the former case a second vector is to be employed for the expression of the guide RNA. CRISPR genome editing vectors are commercially available, for example, from OriGene, Vector Builder or ThermoFisher.

The nucleic acid molecule of the present invention referred to above may also be inserted into vectors such that a translational fusion with another nucleic acid molecule is generated. To this aim, overlap extension PCR can be applied (e.g. Wurch, T., Lestienne, F., and Pauwels, P.J., A modified overlap extension PCR method to create chimeric genes in the absence of restriction enzymes, Biotechn. Techn. 12, 9, Sept. 1998, 653-657). The products arising therefrom are termed fusion proteins and will be described further below. The other nucleic acid molecules may encode a protein which may e.g. increase the solubility and/or facilitate the purification of the protein encoded by the nucleic acid molecule of the invention. Non-limiting examples include pET32, pET41, pET43. The vectors may also contain an additional expressible nucleic acid coding for one or more chaperones to facilitate correct protein folding. Suitable bacterial expression hosts comprise e. g. strains derived from BL21 (such as BL21(DE3), BL21(DE3)PlysS, BL21(DE3)RIL, BL21(DE3)PRARE) or Rosetta^{®}.

For vector modification techniques, see J.F. Sambrook and D.W. Russell, ed., Cold Spring Harbor Laboratory Press, 2001, ISBN-10 0-87969-577-3. Generally, vectors can contain one or more origins of replication (ori) and inheritance systems for cloning or expression, one or more markers for selection in the host, e.g., antibiotic resistance, and one or more expression cassettes. Suitable origins of replication include, for example, the Col E1, the SV40 viral and the M13 origins of replication.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e. g., translation initiation codon, transcriptional termination sequences, promoters, enhancers, and/or insulators), internal ribosomal entry sites (IRES) (Owens et al., (2001), PNAS. 98 (4) 1471-1476) and optionally poly-A signals ensuring termination of transcription and stabilization of the transcript. Additional regulatory elements may include transcriptional as well as translational enhancers, and/or naturally-associated or heterologous promoter regions. The regulatory elements may be native to the endonuclease of the invention or heterologous regulatory elements. Preferably, the nucleic acid molecule of the invention is operably linked to such expression control sequences allowing expression in prokaryotes or eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the nucleic acid molecule of the invention. Such leader sequences are well known in the art. Specifically designed vectors allow the shuttling of DNA between different hosts, such as bacteria-fungal cells or bacteria-animal cells.

Additionally, baculoviral systems or systems based on Vaccinia Virus or Semliki Forest Virus can be used as vectors in eukaryotic expression systems for the nucleic acid molecules of the invention. Expression vectors derived from viruses such as retroviruses, vaccinia virus, adeno-associated virus, herpes viruses, or bovine papilloma virus, may be used for delivery of the nucleic acids or vector into targeted cell populations. Methods which are well known to those skilled in the art can be used to construct recombinant viral vectors; see, for example, the techniques described in Sambrook and D.W. Russell, ed., Cold Spring Harbor Laboratory Press, 2001.

Examples for regulatory elements permitting expression in eukaryotic host cells are promoters, including the promoters as described herein above. Besides elements which are responsible for the initiation of transcription such regulatory elements may also comprise transcription termination signals, such as the SV40-poly-A site or the tk-poly-A site or the SV40, lacZ and AcMNPV polyhedral polyadenylation signals, downstream of the nucleic acid.

The co-transfection with a selectable marker such as kanamycin or ampicillin resistance genes for culturing in *E. coli* and other bacteria allows the identification and isolation of the transfected cells. Selectable markers for mammalian cell culture are the dhfr, gpt, neomycin, hygromycin resistance genes. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS). Using such markers, the cells are grown in selective medium and the cells with the highest resistance are selected.

However, the nucleic acid molecules of the invention as described herein above may also be designed for direct introduction or for introduction via liposomes, phage vectors or viral vectors (e.g. adenoviral or retroviral) into the cell.

The present invention relates in a third aspect to a host cell comprising the nucleic acid molecule of the first aspect or being transformed, transduced or transfected with the vector of the second aspect.

The definitions and preferred embodiments as described herein above apply *mutatis mutandis* to the third aspect, if applicable.

Large amounts of the RNA-guided DNA endonuclease may be produced by said host cell, wherein the isolated nucleotide sequence encoding the RNA-guided DNA endonuclease is inserted into an appropriate vector or expression vector before insertion into the host. The vector or expression vector is introduced into an appropriate host cell, which preferably can be grown in large quantities, and the RNA-guided DNA endonuclease is purified from the host cells or the culture media.

The host cells may also be used to supply the RNA-guided DNA endonuclease of the invention without requiring purification of the RNA-guided DNA endonuclease (see Yuan, Y.; Wang, S.; Song, Z.; and Gao, R., Immobilization of an L-aminoacylase-producing strain of Aspergillus oryzae into gelatin pellets and its application in the resolution of D,L-methionine, Biotechnol Appl. Biochem. (2002). 35:107-113). The RNA-guided DNA endonuclease of the invention may be secreted by host cells. Those skilled in the field of molecular biology will understand that any of a wide variety of expression systems may be used to provide the RNA-guided DNA endonuclease. The precise host cell used is not critical to the invention, so long as the host cells produce the RNA-guided DNA endonuclease when grown under suitable growth conditions.

Host cells into which vectors containing the nucleic acid molecule of the invention can be cloned are used for replicating and isolating a sufficient quantity of the recombinant enzyme. The methods used for this purpose are well known to the skilled person (Sambrook and D.W. Russell, ed., Cold Spring Harbor Laboratory Press, 2001).

The expression of the RNA-guided DNA endonuclease may not only be used to produce the RNA-guided DNA endonuclease in a host cell, but its expression may also be used to edit the genome of the host cell. In such a case the host cell also comprises a guide RNA. Vectors that can be used for CRISPR genome editing have been discussed herein above.

In accordance with a preferred aspect of the third aspect of the invention the host cell is a eukaryotic cell or a prokaryotic cell and is preferably a plant cell or an animal cell.

The host cell can be a eukaryotic cell that does not naturally include a gene encoding any one of BMC01 - BMC15, and can be, for example, be the cell of a fungus, algae, plant, or animal, wherein the animal can be an avian, reptile, amphibian, fish, cephalopod, crustacean, insect, arachnid, marsupial, or mammalian. The gene encoding any one of BMC01 - BMC15 that is non-native with respect to the host cell can be operably linked to a regulatory element, such as a promoter. The promoter can be native to the host organism or can be a promoter of another species. A construct for expressing any one of BMC01 - BMC15 in a heterologous host cell, such as a eukaryotic cell, can optionally further include a transcriptional terminator. The gene encoding any one of BMC01 - BMC15 can optionally be codon optimized for the host species, can optionally include one or more introns, and can optionally include one or more peptide tag sequences, one or more nuclear localisation sequences (NLSs) and/or one or more linkers or engineered cleavage sites (e.g. a 2a sequence). In various embodiments a host cell can include any of the engineered BMC01 - BMC15 CRISPR systems disclosed above, where the nucleic acid sequence encoding the effector is present in the cell prior to introduction of a guide RNA. In other embodiments, the cell that is engineered to include a gene for expressing BMC01 - BMC15 polypeptide can further include a polynucleotide encoding a guide RNA (e.g., a guide RNA) that is operably linked to a regulatory element.

The cell or organism can be a prokaryotic cell that does not naturally include a gene encoding any one of BMC01 - BMC15. Suitable prokaryotic host cells comprise e.g. bacteria of the species Escherichia, such as strains derived from *E. coli* BL21 (e.g. BL21(DE3), BL21(DE3)PlysS, BL21(DE3)RIL, BL21(DE3)PRARE, BL21 codon plus, BL21(DE3) codon plus), Rosetta^{®}, XL1 Blue, NM522, JM101, JM109, JM105, RR1, DH5α, TOP 10, HB101 or MM294. Further suitable bacterial host cells are, but not limited to, *Streptomyces, Pseudomonas,* such as *Pseudomonas putida, Corynebacterium, such as C. glutamicum, Lactobacillus, such as L. salivarius* or *Bacillus* such as *Bacillus subtilis.*

In general, a eukaryotic host cell is preferred over a prokaryotic host cell.

The eukaryotic cell can be a yeast, fungus, amoebae, insect, vertebrate (e.g. mammalian) or plant cells.

Yeasts cells can be, for example, from *Saccharomyces cerevisiae, Ogataea angusta, Kluyveromyces sp. such as K. marxianus* or *K. lactis* or *Pichia sp.* such as *P. pastoris,* insect cells such as Drosophila S2 or Spodoptera Sf9 cells, plant cells, or fungi *cells, preferably of the* family *Trichocomaceae,* more preferably of the genus *Aspergillus, Penicillium* or *Trichoderma, or of the family Ustilaginaceae,* preferably *Ustilago sp..*

Plant host cells that may be used include monocots and dicots (i.e., monocotyledonous and dicotyledonous, respectively), preferably crop plant cells and tobacco cells. Crop plants include cereals (e.g. maize, wheat, rice), legumes (e.g. soybean, pea, and alfalfa), fruit and vegetables (e.g. lettuce, tomato, and potato). Examples of plant species of interest include, but are not limited to, corn (Zea mays), Brassica sp. (e.g., B. napus, B. rapa, B. juncea), particularly those Brassica species useful as sources of seed oil, alfalfa (Medicago sativa), rice (Oryza sativa), rye (Secale cereale), sorghum (Sorghum bicolor, Sorghum vulgare), camelina (Camelina sativa), millet (e.g., pearl millet (Pennisetum glaucum), proso millet (Panicum miliaceum), foxtail millet (Setaria italica), finger millet (Eleusine coracana)), sunflower (Helianthus annuus), quinoa (Chenopodium quinoa), chicory (Cichorium intybus), lettuce (Lactuca sativa), safflower (Carthamus tinctorius), wheat (Triticum aestivum), soybean (Glycine max), tobacco (Nicotiana tabacum), potato (Solanum tuberosum), peanuts (Arachis hypogaea), cotton (Gossypium barbadense, Gossypium hirsutum), sweet potato (Ipomoea batatus), cassava (Manihot esculenta), coffee (Coffea spp.), coconut (Cocos nucifera), pineapple (Ananas comosus), citrus trees (Citrus spp.), cocoa (Theobroma cacao), tea (Camellia sinensis), banana (Musa spp.), avocado (Persea americana), fig (Ficus casica), guava (Psidium guajava), mango (Mangifera indica), olive (Olea europaea), papaya (Carica papaya), cashew (Anacardium occidentale), macadamia (Macadamia integrifolia), almond (Prunus amygdalus), sugar beets (Beta vulgaris), sugarcane (Saccharum spp.), oil palm (Elaeis guineensis), poplar (Populus spp.), eucalyptus (Eucalyptus spp.), oats (Avena sativa), barley (Hordeum vulgare), vegetables, ornamentals, and conifers.

Mammalian host cells that could be used include human Hela, HEK293, H9 and Jurkat cells, mouse NIH3T3 and C127 cells, COS 1, COS 7 and CV1, quail QC1-3 cells, mouse L cells, Bowes melanoma cells and Chinese hamster ovary (CHO) cells.

The present invention relates in a fourth aspect to a plant, seed or a part of a plant, said part of a plant no being a single plant cell, or an animal comprising the nucleic acid molecule of the first aspect or being transformed, transduced or transfected with the vector of the second aspect.

The definitions and preferred embodiments as described herein above apply *mutatis mutandis* to the fourth aspect, if applicable.

The plant or a part thereof may be a monocotyledonous plant or part thereof or a dicotyledonous plant or part thereof. The dicotyledonous plant is preferably a tobacco plant or part thereof. Likewise the seed may be a monocotyledonous or dicotyledonous plant seed and is preferably tobacco plant seed.

Preferred examples of crop plants have been described herein above. Non-limiting examples of a part of a plant is a leave, stem, or root.

The animal is preferably a mammal and most preferably a non-human mammal. The mammal can be, for example, a mouse, rat, hamster, cat, dog, horse, swine, cattle, monkey, ape, etc.

By the expression of the nucleic acid molecule of the first aspect in a plant, seed or a part of a plant or an animal along with a guide RNA the genome of the host may be edited. The genome may be edited, for example, in order to introduce a targeted gene mutation, for gene therapy, for a creating chromosome rearrangement, for studying gene function, for the production of a transgenic organism, for endogenous gene labeling or for targeted transgene addition.

The present invention relates in a fifth aspect to a method of producing an RNA-guided DNA endonuclease comprising culturing the host cell of the third aspect and isolating the RNA-guided DNA endonuclease produced.

The definitions and preferred embodiments as described herein above apply *mutatis mutandis* to the fifth aspect, if applicable.

Suitable conditions for culturing a prokaryotic or eukaryotic host are well known to the person skilled in the art. In general, suitable conditions for culturing bacteria are growing them under aeration in Luria Bertani (LB) medium. To increase the yield and the solubility of the expression product, the medium can be buffered or supplemented with suitable additives known to enhance or facilitate both. *E. coli* can be cultured from 4 to about 37 °C, the exact temperature or sequence of temperatures depends on the molecule to be overexpressed.

In general, *Aspergillus sp.* may be grown on Sabouraud dextrose agar, or potato dextrose agar at about to 10°C to about 40°C, and preferably at about 25°C. Suitable conditions for yeast cultures are known, for example from Guthrie and Fink, "Guide to Yeast Genetics and Molecular Cell Biology" (2002); Academic Press Inc.. The skilled person is also aware of all these conditions and may further adapt these conditions to the needs of a particular host species and the requirements of the polypeptide expressed. In case an inducible promoter controls the nucleic acid of the invention in the vector present in the host cell, expression of the polypeptide can be induced by addition of an appropriate inducing agent. Suitable expression protocols and strategies are known to the skilled person.

Depending on the cell type and its specific requirements, mammalian cell culture can e.g. be carried out in RPMI or DMEM medium containing 10% (v/v) FCS, 2mM L-glutamine and 100 U/ml penicillin/streptomycin. The cells can be kept at 37 °C in a 5% CO₂, water saturated atmosphere. Suitable expression protocols for eukaryotic cells are well known to the skilled person and can be retrieved e.g. from Sambrook, 2001.

Methods for the isolation of the produced RNA-guided DNA endonuclease are well-known in the art and comprise without limitation method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, in Sambrook, 2001.

The step of protein isolation is preferably a step of protein purification. Protein purification in accordance with the invention specifies a process or a series of processes intended to further isolate the polypeptide of the invention from a complex mixture, preferably to homogeneity. Purification steps, for example, exploit differences in protein size, physico-chemical properties and binding affinity. For example, proteins may be purified according to their isoelectric points by running them through a pH graded gel or an ion exchange column. Further, proteins may be separated according to their size or molecular weight via size exclusion chromatography or by SDS-PAGE (sodium dodecyl sulfate-polyacrylamide gel electrophoresis) analysis. In the art, proteins are often purified by using 2D-PAGE and are then further analysed by peptide mass fingerprinting to establish the protein identity. This is useful for scientific purposes and the detection limits for protein are very low and nanogram amounts of protein are sufficient for their analysis. Proteins may also be purified by polarity/hydrophobicity via high performance liquid chromatography or reversed-phase chromatography. Thus, methods for protein purification are well known to the skilled person.

The present invention relates in a sixth aspect to an RNA-guided DNA endonuclease encoded by the nucleic acid molecule of the first aspect.

The definitions and preferred embodiments as described herein above apply *mutatis mutandis* to the sixth aspect, if applicable.

The amino acid sequences of SEQ ID NOs 1- 15 are particularly preferred examples of the RNA-guided DNA endonuclease of the invention.

The RNA-guided DNA endonuclease of the sixth aspect of the invention may also be a fusion protein, wherein the amino acid sequence of the RNA-guided DNA endonuclease is fused to a fusion partner. The fusion may be a direct fusion or a fusion via a linker. The linker is preferably a peptide, such as a GS-linker.

The fusion partner can be located at the N-terminus, the C-terminus, at both termini, or in an internal location of the RNA-guided DNA endonuclease polypeptide, preferably at the N- or C-terminus.

The fusion partner is preferably a nuclear localization signal (NLS), a cell-penetrating domain, a plastid targeting signal, a mitochondrial targeting signal peptide, a signal peptide targeting both plastids and mitochondria, a marker domain, a tag (such as a purification tag), a DNA modifying enzyme or a transactivation domain.

DNA modifying enzymes may modify the DNA by phosphorylation, dephosphorylation of blunting DNA, wherein blunting refers the digestion single-stranded overhangs. Non-limiting examples of dephosphorylation enzymes are the Shrimp Alkaline Phosphatase (rSAP), Quick CIP Phosphatase and Antarctic Phosphatase. Non-limiting examples of phosphorylation enzymes are polynucleotide kinases, such as T4 PNK. Non-limiting examples of blunting enzymes are the DNA Polymerase I Large (Klenow) Fragment, T4 DNA Polymerase or Mung Bean Nuclease.

Transactivation domains (or trans-activating domains (TADs)) are transcription factor scaffold domains which contain binding sites for other proteins such as transcription co-regulators. Non-limiting examples are nine-amino-acid transactivation domains (9aaTADs) and Glutamine (Q)-rich TADs.

In general, an NLS comprises a stretch of basic amino acids. Nuclear localization signals are known in the art. The NLS can be at the N- terminus, the C-terminus or both the RNA-guided DNA endonuclease polypeptide according to the invention. For instance, the RNA-guided DNA endonuclease polypeptide according to the invention may comprise about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g., zero or at least one or more NLS at the amino-terminus and zero or at one or more NLS at the carboxy-terminus). When more than one NLS is present, each may be selected independently of the others, such that a single NLS may be present in more than one copy and/or in combination with one or more other NLSs present in one or more copies. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the N- or C- terminus. The RNA-guided DNA endonuclease polypeptide sequence and the NLS may in some embodiments be fused with a linker between 1 to about 20 amino acids in length.

Non-limiting examples of NLSs include an NLS sequence derived from: the NLS of the SV40 virus large T-antigen; the NLS from nucleoplasmin (e.g., the nucleoplasmin bipartite NLS); the c-myc NLS; the hRNPAI M9 NLS; the IBB domain from importin-alpha; the NLS sequences of the myoma T protein, the p53 protein; the c-abl IV protein, or influenza virus NS 1 ; the NLS of the Hepatitis virus delta antigen, the Mxl protein; the poly(ADP-ribose) polymerase; and the steroid hormone receptors (human) glucocorticoid. In general, the one or more NLSs are of sufficient strength to drive accumulation of the RNA-guided DNA endonuclease polypeptide according to the invention in a detectable amount in the nucleus of a eukaryotic cell.

Plastid, mitochondrial, and dual-targeting signal peptide localization signals are also known in the art (see, e.g., Nassoury and Morse (2005) Biochim Biophys Acta 1743:5-19; Kunze and Berger (2015) Front Physiol 6:259; Herrmann and Neupert (2003) IUBMB Life 55:219-225; Soil (2002) Curr Opin Plant Biol 5:529-535; Carrie and Small (2013) Biochim Biophys Acta 1833:253-259; Carrie et al. (2009) FEBS J 276: 1187-1195; Silva-Filho (2003) Curr Opin Plant Biol 6:589-595; Peeters and Small (2001) Biochim Biophys Acta 1541:54-63; Murcha et al. (2014) Exp Bot 65:6301-6335; Mackenzie (2005) Trends Cell Biol 15:548-554; Glaser et al. (1998) Plant Mol Biol 38:311-338).

Non-limiting examples of marker domains include fluorescent proteins, purification tags, and epitope tags. In certain embodiments, the marker domain can be a fluorescent protein. Non-limiting examples of suitable fluorescent proteins include green fluorescent proteins (e.g., GFP, GFP-2, tagGFP, turboGFP, EGFP, Emerald, Azami Green, Monomeric Azami Green, CopGFP, AceGFP, ZsGreenl), yellow fluorescent proteins (e.g. YFP, EYFP, Citrine, Venus, YPet, PhiYFP, ZsYellowl), blue fluorescent proteins (e.g. EBFP, EBFP2, Azurite, mKalamal, GFPuv, Sapphire, T-sapphire), cyan fluorescent proteins (e.g. ECFP, Cerulean, CyPet, AmCyanl, Midoriishi-Cyan), red fluorescent proteins (mKate, mKate2, mPlum, DsRed monomer, mCherry, mRFPI, DsRed- Express, DsRed2, DsRed-Monomer, HcRed-Tandem, HcRedl, AsRed2, eqFP611, mRasberry, mStrawberry, Jred), and orange fluorescent proteins (mOrange, mKO, Kusabira-Orange).

A tag is short amino acid sequence that allows the identification of the RNA-guided DNA endonuclease polypeptide according to the invention in a mixture of polypeptides. Hence, the tag is preferably a purification tag. Non-limiting examples of a purification tag are a His-tag (e.g. His-6-tag). a GST-tag, DHFR-tag and a CBP-tag, A review of known purification tags can be found in Kimple et al. (2015), Curr Protoc Protein Sci. 2013; 73: Unit-9.9.

The present invention relates in a seventh aspect to a composition comprising the nucleic acid molecule of the first aspect, the vector of the second aspect, the host cell of the third aspect, the plant, seed, part of a cell or animal of the fourth aspect, the RNA-guided DNA endonuclease of the sixth aspect or a combination thereof.

The definitions and preferred embodiments as described herein above apply *mutatis mutandis* to the seventh aspect, if applicable.

The term "composition" as used herein refers to a composition comprising at least one of the nucleic acid molecule of the first aspect, the vector of the second aspect, the host cell of the third aspect, the plant, seed, part of a cell or animal of the fourth aspect, the RNA-guided DNA endonuclease of the sixth aspect or a combination thereof which are also collectively referred in the following as compounds.

In accordance with a preferred embodiment of the seventh aspect the composition is a pharmaceutical composition or a diagnostic composition.

In accordance with the present invention, the term "pharmaceutical composition" relates to a composition for administration to a patient, preferably a human patient. The pharmaceutical composition of the invention comprises at least one of the compounds recited above. It may, optionally, comprise further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). The pharmaceutical composition of the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by conventional methods. These pharmaceutical compositions may be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g of the active compound per day. However, a more preferred dosage might be in the range of 0.01 mg to 100 mg, even more preferably 0.01 mg to 50 mg and most preferably 0.01 mg to 10 mg per day. The length of treatment needed to observe changes and the interval following treatment for responses to occur vary depending on the desired effect. The particular amounts may be determined by conventional tests which are well known to the person skilled in the art.

The pharmaceutical composition may be used, for example, to treat or prevent a pathogenic disease, such as a viral or bacterial disease. For instance, the RNA-guided DNA endonuclease of the sixth aspect may be used together with gRNA targeting the genome of the pathogen thereby modifying the genome of the pathogen, such that the disease being caused by the pathogen is prevented or treated.

The pharmaceutical composition may also be used, for example, to treat or prevent a microbiome imbalance. An imbalance in the microbiome can occur, for example, because of an overuse of antibiotics, which may cause an overgrowth of pathogenic bacteria and yeast.

A "diagnostic composition" relates to composition which is suitable to detect a disease in subject, both infectious and non-infectious disease. The diagnostic composition may in particular comprise a marker portion as described herein above in connection with the fusion protein of the invention being attached to ssDNA strands, so that when RNA-guided DNA endonuclease polypeptide according to the invention cuts the ssDNA, it activates the reporter, causing it to fluoresce or change colour, thus enabling visual detection of the specific disease nucleic marker. The diagnostic composition may be applied to a body fluid sample, such as a blood, urine, or saliva.

The present invention relates in a eighth aspect to the nucleic acid molecule of the first aspect, the vector of the second aspect, the host cell of the third aspect, the plant, seed, part of a cell or animal of the fourth aspect, the RNA-guided DNA endonuclease of the sixth aspect or a combination thereof for use in the treatment of a disease in a subject or a plant by modifying a nucleotide sequence at a target site in the genome of the subject or plant.

Also described is method of treating or preventing a disease in a subject or a plant comprising modifying a nucleotide sequence at a target site in the genome of the subject or plant by the nucleic acid molecule of the first aspect, the vector of the second aspect, the host cell of the third aspect, the plant, seed, part of a cell or animal of the fourth aspect, the RNA-guided DNA endonuclease of the sixth aspect or a combination thereof.

The definitions and preferred embodiments as described herein above apply *mutatis mutandis* to the eighth aspect, if applicable.

The modification of a nucleotide sequence at a target site in the genome of the subject or plant is in accordance with the invention a genome editing by the CRISPR technology and in particular by the novel RNA-guided DNA endonucleases as provided herewith which is to be used in combination with a proper gRNA and optionally a repair substrate as described herein below in order to determine the target side of the genome modification.

Genome editing (also known as genome engineering) is a type of genetic engineering in which a target site, preferably a gene of interest is inserted, deleted, modified or replaced in the genome of the cell. The target site, preferably the gene of interest can be in the genome but may also be in the mitochondrial DNA (animal cells) or chloroplast DNA (plant cells). Genome editing may result in a loss-of-function mutation or a gain-of-function mutation in the genome of the cell. A loss-of-function mutation (also called inactivating mutation) results in the gene of interest having less or no function (being partially or wholly inactivated). When the allele has a complete loss of function (wholly inactivated) this is also called herein a (gene) knock-out. A gene knock-out may be achieved by inserting, deleting, modifying or replacing one or more nucleotides of a gene. A gain-of-function mutation (also called activating mutation) may change the gene of interest such that its effect becomes stronger (enhanced activation) or even is superseded by a different (e.g. abnormal) function. A gain-of-function mutation may also introduce a new function or effect into a cell which the cell did not have before. In this context the new gene may be added to the genome of the cell (insertion) or may replace a gene within the genome. A gain-of-function mutation introducing such a new function or effect is also called gene knock-in. Genome editing may also result in the up- or down-regulation of one or more genes. By targeting DNA sites which are responsible for the regulation of the expression of a gene (e.g. a promoter region or a gene encoding transcription factor) the expression of genes can be up- or down-regulated by CRISPR technology. Further details on the mode of action of the CRISPR technology will be provided herein below.

Since its discovery, the CRISPR technology has been increasingly applied to therapeutic genome editing. Employment of several viral and non-viral vectors has enabled efficient delivery of the CRISPR system to target cells or tissues. In addition, the CRISPR system is able to modulate the target gene's expression in various ways, such as mutagenesis, gene integration, epigenome regulation, chromosomal rearrangement, base editing and mRNA editing (for review Le and Kim (2019), Hum Genet; 138(6):563-590).

The modification of a nucleotide sequence at a target site in the genome of the subject is preferably a gene therapy. Gene therapy is based on the principle of the genetic manipulation of nucleotide sequence at a target site for treating and preventing a disease, in particular a human disease.

In clinical trials of the CRISPR technology, scientists are using the CRISPR technology to combat cancer and blood disorders in humans. In these trials, some cells are removed from the subject to be treated, the DNA is genome-edited and then the genome edited cells are put back into the subject, said cells now being armed to fight the disease to be treated.

The present invention relates in a ninth aspect to a method of modifying a nucleotide sequence at a target site in the genome of a cell comprising introducing into said cell (i) a DNA-targeting RNA or a DNA polynucleotide encoding a DNA-targeting RNA, wherein the DNA-targeting RNA comprises: (a) a first segment comprising a nucleotide sequence that is complementary to a sequence in the target DNA; and (b) a second segment that interacts with the RNA-guided DNA endonuclease of the sixth aspect; and (ii) the RNA-guided DNA endonuclease of the sixth aspect, or the nucleic acid molecule encoding an RNA-guided DNA endonuclease of the first aspect, or the vector of the second aspect, wherein the RNA-guided DNA endonuclease comprises (a) an RNA-binding portion that interacts with the DNA-targeting RNA and (b) an activity portion that exhibits site-directed enzymatic activity.

Accordingly, the present invention also relates to a composition (e.g. a pharmaceutical or a diagnostic composition) comprising (i) a DNA-targeting RNA or a DNA polynucleotide encoding a DNA-targeting RNA, wherein the DNA-targeting RNA comprises: (a) a first segment comprising a nucleotide sequence that is complementary to a sequence in the target DNA; and (b) a second segment that interacts with the RNA-guided DNA endonuclease of the sixth aspect; and (ii) the RNA-guided DNA endonuclease of the sixth aspect, or the nucleic acid molecule encoding an RNA-guided DNA endonuclease of the first aspect, or the vector of the second aspect, wherein the RNA-guided DNA endonuclease comprises (a) an RNA-binding portion that interacts with the DNA-targeting RNA and (b) an activity portion that exhibits site-directed enzymatic activity.

The definitions and preferred embodiments as described herein above apply *mutatis mutandis* to the ninth aspect, if applicable.

The DNA-targeting RNA comprises a first segment comprising a nucleotide sequence that is complementary to a sequence in the target DNA and a second segment that interacts with the RNA-guided DNA endonuclease. As discussed herein above, the nucleotide sequence that is complementary to a sequence in the target DNA defines the target specificity of the RNA-guided DNA endonuclease. As also discussed herein above, the DNA-targeting RNA binds to the RNA-guided DNA endonuclease, whereby a complex is formed. The second segment interacts with the RNA-guided DNA endonuclease and is responsible for the formation of the complex. The second segment that interacts with the RNA-guided DNA endonuclease of the sixth aspect preferably comprises or consists of SEQ ID NO: 31 and more preferably of SEQ ID NO: 32. SEQ ID NO: 31 is a consensus sequence of the second segment of Type V Class 2 CRISPR nucleases. In Type V Class 2 CRISPR nucleases the second segment is also known as 5' handle. SEQ ID NO: 32 is the second segment of any one of BMC01 - BMC15.

The RNA-guided DNA endonuclease comprises as first segment being an RNA-binding portion that interacts with the DNA-targeting RNA and as a second segment being an activity portion that exhibits site-directed enzymatic activity. The first segment interacts with the DNA-targeting RNA and is responsible for the formation of the discussed complex. The second segment harbours the endonuclease domain, which preferably comprises a RuvC domain.

As also discussed herein above, the DNA-targeting RNA is the guide RNA. The guide RNA may either be directly introduced into the cells or as a DNA polynucleotide encoding the DNA-targeting RNA. In the latter case, the DNA encoding the guide RNA is generally operably linked to one or more promoter sequences for expression of the guide RNA. For example, the RNA coding sequence can be operably linked to a promoter sequence that is recognized by RNA polymerase III (Pol III) or RNA polymerase II (Pol II). The DNA polynucleotide encoding the DNA-targeting RNA is preferably a vector. Many single gRNA empty vectors (with and without the CRISPR endonuclease) are available in the art. Also several empty multiplex gRNA vectors are available that can be used to express multiple gRNAs from a single plasmid (with or without the expression of the CRISPR endonuclease). The DNA polynucleotide encodes the DNA-targeting RNA in expressible form.

Likewise, the RNA-guided DNA endonuclease may either be directly introduced into the cells or as a nucleic acid molecule encoding the RNA-guided DNA endonuclease, the latter being preferably a vector of the second aspect. The DNA polynucleotide encodes the RNA-guided DNA endonuclease in expressible form.

As is discussed in greater detail herein above, the RNA-guided DNA endonuclease and the DNA-targeting RNA may also be encoded by the same DNA polynucleotide, such as an all-in one CRISPR-cas vector.

The term "in expressible form" means that the one or more DNA polynucleotides encoding the RNA-guided DNA endonuclease and the DNA-targeting RNA are in a form that ensures that the DNA-targeting RNA is transcribed and that the RNA-guided DNA endonuclease is transcribed and translated into the active enzyme in the cells.

In accordance with a preferred embodiment of the ninth aspect of the invention in case the RNA-guided DNA endonuclease and the DNA-targeting RNA are directly introduced into the cell they are introduced in the form of a ribonucleoprotein complex (RNP).

RNPs are assembled *in vitro* and can be delivered to the cell by methods known in the art, for example, electroporation or lipofection. RNPs are capable to cleave the target site with comparable efficacy as nucleic acid-based (e.g. vector-based) RNA-guided DNA endonucleases (Kim et al. (2014), Genome Research 24(6):1012-1019).

Means for introducing proteins (or peptides) or RNPs into living cells are known in the art and comprise but are not limited to microinjection, electroporation, lipofection (using liposomes), nanoparticle-based delivery, and protein transduction. Any one of these methods may be used.

A liposome used for lipofection is a small vesicle, composed of the same material as a cell membrane (i.e., normally a lipid bilayer e.g. made of phospholipids), which can be filled with one or more protein(s) (e.g. Torchilin VP. (2006), Adv Drug Deliv Rev., 58(14):1532-55). To deliver a protein or RNP into a cell, the lipid bilayer of the liposome can fuse with the lipid bilayer of the cell membrane, thereby delivering the contained protein into the cell. It is preferred that the liposomes used in accordance with invention are composed of cationic lipids. The cationic liposome strategy has been applied successfully to protein delivery (Zelphati et al. (2001). J. Biol. Chem. 276, 35103-35110). As known in the art, the exact composition and/or mixture of cationic lipids used can be altered, depending upon the protein(s) of interest and the cell type used (Felgner et al. (1994). J. Biol. Chem. 269, 2550-2561). Nanoparticle-based delivery of Cas9 ribonucleoprotein and donor DNA for the induction of homology-directed DNA repair is, for example, described in Lee et al. (2017), Nature Biomedical Engineering, 1:889-90.

Protein transduction specifies the internalisation of proteins into the cell from the external environment (Ford et al (2001), Gene Therapy, 8:1-4). This method relies on the inherent property of a small number of proteins and peptides (preferably 10 to 16 amino acids long) to penetrate the cell membrane. The transducing property of these molecules can be conferred upon proteins which are expressed as fusions with them and thus offer, for example, an alternative to gene therapy for the delivery of therapeutic proteins into target cells. Commonly used proteins or peptides being able to penetrate the cell membrane are, for example; the antennapedia peptide, the herpes simplex virus VP22 protein, HIV TAT protein transduction domain, peptides derived from neurotransmitters or hormones, or a 9xArg-tag.

Microinjection and electroporation are well known in the art and the skilled person knows how to perform these methods. Microinjection refers to the process of using a glass micropipette to introduce substances at a microscopic or borderline macroscopic level into a single living cell. Electroporation is a significant increase in the electrical conductivity and permeability of the cell plasma membrane caused by an externally applied electrical field. By increasing permeability, protein (or peptides or nucleic acid sequences) can be introduced into the living cell.

The RNA-guided DNA endonuclease may be introduced into the cells as an active enzyme or as a proenzyme. In the latter case the RNA-guided DNA endonuclease is biochemically changed within the cells (for example by a hydrolysis reaction revealing the active site, or changing the configuration to reveal the active site), so that the proenzyme becomes an active enzyme.

Means and methods for the introduction of nucleic acid molecule(s) and DNA-targeting RNA into cells are likewise known in the art and these methods encompass transducing or transfecting cells.

Transduction is the process by which foreign DNA is introduced into a cell by a virus or viral vector. Transduction is a common tool used by molecular biologists to stably introduce a foreign gene into a host cell's genome. Generally, a plasmid is constructed in which the genes to be transferred are flanked by viral sequences that are used by viral proteins to recognize and package the viral genome into viral particles. This plasmid is inserted (usually by transfection) into a producer cell together with other plasmids (DNA constructs) that carry the viral genes required for formation of infectious virions. In these producer cells, the viral proteins expressed by these packaging constructs bind the sequences on the DNA/RNA (depending on the type of viral vector) to be transferred and insert it into viral particles. For safety, none of the plasmids used contains all the sequences required for virus formation, so that simultaneous transfection of multiple plasmids is required to get infectious virions. Moreover, only the plasmid carrying the sequences to be transferred contains signals that allow the genetic materials to be packaged in virions, so that none of the genes encoding viral proteins are packaged. Viruses collected from these cells are then applied to the cells to be altered. The initial stages of these infections mimic infection with natural viruses and lead to expression of the genes transferred and (in the case of lentivirus/retrovirus vectors) insertion of the DNA to be transferred into the cellular genome. However, since the transferred genetic material does not encode any of the viral genes, these infections do not generate new viruses (the viruses are "replication-deficient"). In the present case transduction may be used to generate cells that comprise the RNA-guided DNA endonuclease in their genome in expressible form.

Transfection is the process of deliberately introducing naked or purified nucleic acids or purified proteins or assembled ribonucleoprotein complexes into cells. Transfection is generally a non-viral based method.

Transfection may be a chemical-based transfection. Chemical-based transfection can be divided into several kinds: transfection using cyclodextrin, polymers, liposomes, or nanoparticles. One of the cheapest methods uses calcium phosphate. HEPES-buffered saline solution (HeBS) containing phosphate ions are combined with a calcium chloride solution containing the DNA to be transfected. When the two are combined, a fine precipitate of the positively charged calcium and the negatively charged phosphate will form, binding the DNA to be transfected on its surface. The suspension of the precipitate is then added to the cells to be transfected (usually a cell culture grown in a monolayer). By a process not entirely understood, the cells take up some of the precipitate, and with it, the DNA. This process has been a preferred method of identifying many oncogenes. Other methods use highly branched organic compounds, so-called dendrimers, to bind the DNA and transfer it into the cell. Another method is the use of cationic polymers such as DEAE-dextran or polyethylenimine (PEI). The negatively charged DNA binds to the polycation and the complex is taken up by the cell via endocytosis. Lipofection (or liposome transfection) is a technique used to inject genetic material into a cell by means of liposomes, which are vesicles that can easily merge with the cell membrane since they are both made of a phospholipid bilayer, as mentioned above. Lipofection generally uses a positively charged (cationic) lipid (cationic liposomes or mixtures) to form an aggregate with the negatively charged (anionic) genetic material. This transfection technology performs the same tasks in terms of transfer into cells as other biochemical procedures utilizing polymers, DEAE-dextran, calcium phosphate, and electroporation. The efficiency of lipofection can be improved by treating transfected cells with a mild heat shock. Fugene is a series of widely used proprietary non-liposomal transfection reagents capable of directly transfecting a wide variety of cells with high efficiency and low toxicity.

Transfection may also be a non-chemical method. Electroporation (gene electrotransfer) is a popular method, where transient increase in the permeability of cell membrane is achieved when the cells are exposed to short pulses of an intense electric field. Cell squeezing enables delivery of molecules into cells via cell membrane deformation. Sonoporation uses high-intensity ultrasound to induce pore formation in cell membranes. This pore formation is attributed mainly to the cavitation of gas bubbles interacting with nearby cell membranes since it is enhanced by the addition of ultrasound contrast agent, a source of cavitation nuclei. Optical transfection is a method where a tiny (~1 µm diameter) hole is transiently generated in the plasma membrane of a cell using a highly focused laser. Protoplast fusion is a technique in which transformed bacterial cells are treated with lysozyme in order to remove the cell wall. Following this, fusogenic agents (e.g., Sendai virus, PEG, electroporation) are used in order to fuse the protoplast carrying the gene of interest with the recipient target cell.

Finally, transfection may be a particle-based method. A direct approach to transfection is the gene gun, where the DNA is coupled to a nanoparticle of an inert solid (commonly gold), which is then "shot" (or particle bombardment) directly into the target cell's nucleus. Hence, the nucleic acid is delivered through membrane penetration at a high velocity, usually connected to microprojectiles. Magnetofection, or magnet-assisted transfection, is a transfection method that uses magnetic force to deliver DNA into target cells. Impalefection is carried out by impaling cells by elongated nanostructures and arrays of such nanostructures such as carbon nanofibers or silicon nanowires which have been functionalized with plasmid DNA.

The method of the ninth aspect of the invention relates to a method for editing (i.e. "mutating") with the RNA-guided DNA endonuclease of the invention a nucleotide sequence at a target site in the genome of a cell. This requires essentially three sequential preconditions: (1) Efficient delivery of the RNA-guided DNA endonuclease-encoding genes or the RNA-guided DNA endonuclease itself into the target cell; (2) efficient expression or presence of the CRISPR-components in the target cell (DNA-targeting RNA and the RNA-guided DNA endonuclease of the sixth aspect); and (3) targeting of the genomic site of interest by CRISPR ribonucleoprotein complexes and repair of the DNA by cell's own repair pathways. Step (3) is automatically carried out in the cell upon the expression of the CRISPR-components in the cell the genome of which is to be edited.

By genome editing a target site may be inserted, deleted, modified (including singe nucleotide polymorphisms (SNPs)) or replaced in the genome of the cell. The target site can be in the coding region of a gene, in an intron of a gene, in a control region of a gene, in a non-coding region between genes, etc. The gene can be a protein coding gene or an RNA coding gene. The gene can be any gene of interest.

In this connection genome editing uses the cell's own repair pathways, including the non-homologous end-joining (NHEJ) or homology directed recombination (HDR) pathway. Once the DNA is cut by the RNA-guided DNA endonuclease, the cell's own DNA repair machinery (NHEJ or HDR) adds or deletes pieces of genetic material, or makes changes to the DNA by replacing an existing segment with a customized DNA sequence. Hence, in the CRISPR-Cas system, the CRISPR nuclease makes a double-stranded break in DNA at a site determined by the short (~20 nucleotide) gRNA which break is then repaired within the cell by NHEJ or HDR. It is preferred that genome editing uses NHEJ. In a different embodiment, it is preferred that genome editing uses HDR.

NHEJ uses a variety of enzymes to directly join the DNA ends in a double-strand break. In contrast, in HDR, a homologous sequence is utilized as a template for the regeneration of missing DNA sequence at the break point. NHEJ is the canonical homology-independent pathway as it involves the alignment of only one to a few complementary bases at most for the re-ligation of two ends, whereas HDR uses longer stretches of sequence homology to repair DNA lesions.

The natural properties of these pathways form the very basis of RNA-guided DNA endonuclease-based genome editing. NHEJ is error-prone, and has been shown to cause mutations at the repair site. Thus, if one is able to create a double strand break (DSB) at a desired gene in multiple samples, it is very likely that mutations will be generated at that site in some of the treatments because of errors created by the NHEJ infidelity. On the other hand, the dependency of HDR on a homologous sequence to repair DSBs can be exploited by inserting a desired sequence within a sequence that is homologous to the flanking sequences of a DSB which, when used as a template by the HDR system, would lead to the creation of the desired change within the genomic region of interest. Despite the distinct mechanisms, the concept of the HDR based gene editing is in a way similar to that of homologous recombination based gene targeting. So based on these principles if one is able to create a DSB at a specific location within the genome, then the cell's own repair systems will help in creating the desired mutations.

The homologous sequence template for HDR is also referred to herein as "repair template".

Hence, by modifying a nucleotide sequence at a target site in the genome of a cell according to the ninth aspect of the invention a gene may be knocked-out (by introducing per-mature stop codon) or knocked-in (via the repair substrate). It is likewise possible to alter the expression of a gene by the method of the ninth aspect of the invention. For instance, the target site in the genome may be a promoter region changing the promoter region may increase or decrease the expression of the gene being controlled via the target promoter region.

Hence, in accordance with a preferred embodiment of the ninth aspect the method further comprises the introduction of a repair substrate into said cell.

The repair substrate may either be directly introduced into the cell or as a nucleic acid molecule, preferably a vector encoding the repair substrate in expressible from. The use of a repair substrate for correcting a mutant beta-glucuronidase in wheat embryo is illustrated in Example 3.

The designs and structures of repair templates being suitable for HDR are known in the art. HDR is error-free if the repair template is identical to the original DNA sequence at the double-strand break (DSB), or it can introduce very specific mutations into DNA. The three central steps of the HDR pathways are: (1) The 5'-ended DNA strand is resected at the break to create a 3' overhang. This will serve as both a substrate for proteins required for strand invasion and a primer for DNA repair synthesis. (2) The invasive strand can then displace one strand of the homologous DNA duplex and pair with the other. This results in the formation of the hybrid DNA, referred to as the displacement loop (D loop). (3) The recombination intermediates can then be resolved to complete the DNA repair process.

HDR templates used, for example, to introduce mutations or insert new nucleotides or nucleotide sequences into a gene require a certain amount of homology surrounding the target sequence that will be modified. Homology arms can be used that start at the CRISPR-induced DSB. In general, the insertion sites of the modification should be very close to the DSB, ideally less than 10 bp away, if possible. One important point to note is that the CRISPR enzymes may continue to cleave DNA once a DSB is introduced and repaired. As long as the gRNA target site/PAM site remain intact, the CRISPR nuclease will keep cutting and repairing the DNA. This repeated editing may be problematic if a very specific mutation or sequence is to be introduced into a gene of interest. To get around this, the repair template can be designed in such a way that it will ultimately block further CRISPR nuclease targeting after the initial DSB is repaired. Two common ways to block further editing are mutating the PAM sequence or the gRNA seed sequence. When designing a repair template, the size of the intended edit is to be taken into consideration. ssDNA templates (also referred to as ssODNs) are commonly used for smaller modifications. Small insertions/edits may require as little as 30-50 bases for each homology arm, and the best exact number may vary based on the gene of interest. 50-80 base homology arms are commonly used. For example, Richardson et al. (2016). Nat Biotechnol. 34(3):339-44) found that asymmetric homology arms (36 bases distal to the PAM and 91 bases proximal to the PAM) supported HDR efficiencies up to 60%. Due to difficulties that might be associated with creating ssODNs longer than 200 bases, it is preferred to use dsDNA plasmid repair templates for larger insertions such as fluorescent proteins or selection cassettes into a gene of interest. These templates can have homology arms of at least 800 bp. To increase the frequency of HDR edits based on plasmid repair templates, self-cleaving plasmids can be used that contain gRNA target sites flanking the template. When the CRISPR nuclease and the appropriate gRNA(s) are present, the template is liberated from the vector. To avoid plasmid cloning, it is possible to use PCR-generated long dsDNA templates. Moreover, Quadros et al. (2017) Genome Biol.17;18(1):92) developed Easi-CRISPR, a technique that allows making large mutations and to take advantage of the benefits of ssODNs. To create ssODNs longer than 200 bases, RNA encoding the repair template are *in vitro* transcribed and then reverse transcriptase is used to create the complementary ssDNA. Easi-CRISPR works well in mouse knock-in models, increasing editing efficiency from 1-10% with dsDNA to 25-50% with ssODNs. Although HDR efficiency varies across loci and experimental systems, ssODN templates generally provide the highest frequency of HDR edits.

In accordance with a preferred embodiment of the ninth aspect the cell is not the natural host of a gene encoding said RNA-guided DNA endonuclease.

As discussed herein above, the RNA-guided DNA endonucleases of SEQ ID NOs 1 - 15 were isolated from a metagenome library comprising bacteria that cannot be cultured applying standard microbiological conditions. For this reason the exact nature of the bacteria from which SEQ ID NOs 1 - 15 were isolated is not known.

Hence, the cell that is not the natural host of any one of SEQ ID NOs 1 - 15 may be any bacterial cell from a bacterial strain that can be cultured or any eukaryotic cell. Such manipulated cells do not exist in nature.

In accordance with another preferred embodiment of the ninth aspect the cell is a eukaryotic cell, preferably a plant cell or animal cell.

Eukaryotic cells, plant cells and animal cells as well as eukaryotes, plants and animals from which cells may be obtained including preferred examples thereof have been described herein above in connection with the third and fourth aspect of the invention.

These cells may likewise be used in connection with the ninth aspect of the invention.

In accordance with a more preferred embodiment of the ninth aspect the method further comprises culturing the plant cell or animal cell to produce a plant or animal under conditions in which the RNA-guided DNA endonuclease is expressed and cleaves the nucleotide sequence at the target site to produce a modified nucleotide sequence; and selecting a plant or animal comprising said modified nucleotide sequence.

In this connection the cell(s) into which the components of the CRISPR-Cas system are to be introduced has/have to be a totipotent stem cell or a germ line cell (oocyte and/or sperm) or a collection of stem cells being capable of developing into a complete plant or animal. Means and method for culturing such cell(s) in order to produce a plant or animal are known in the art (see for example, https://www.stembook.org/node/720).

Unless otherwise defined, all technical and scientific terms used herein have the same meaning as commonly understood by one of ordinary skill in the art to which this invention belongs. In case of conflict, the patent specification including definitions, will prevail.

The present invention relates in a tenth aspect to modified cells that have been produced by the method according to the ninth aspect of the invention for use in treatment of a disease in a subject.

The modified cells are preferably modified T lymphocytes and the disease to be treated is preferably cancer (Stadtmauer et al., Science 28 Feb 2020:Vol. 367, Issue 6481, eaba7365).

The cells to be modified by the method of the ninth aspect of the invention are preferably obtained from the subject to be treatment and then the modified cells are used in accordance with the tenth aspect of the invention.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

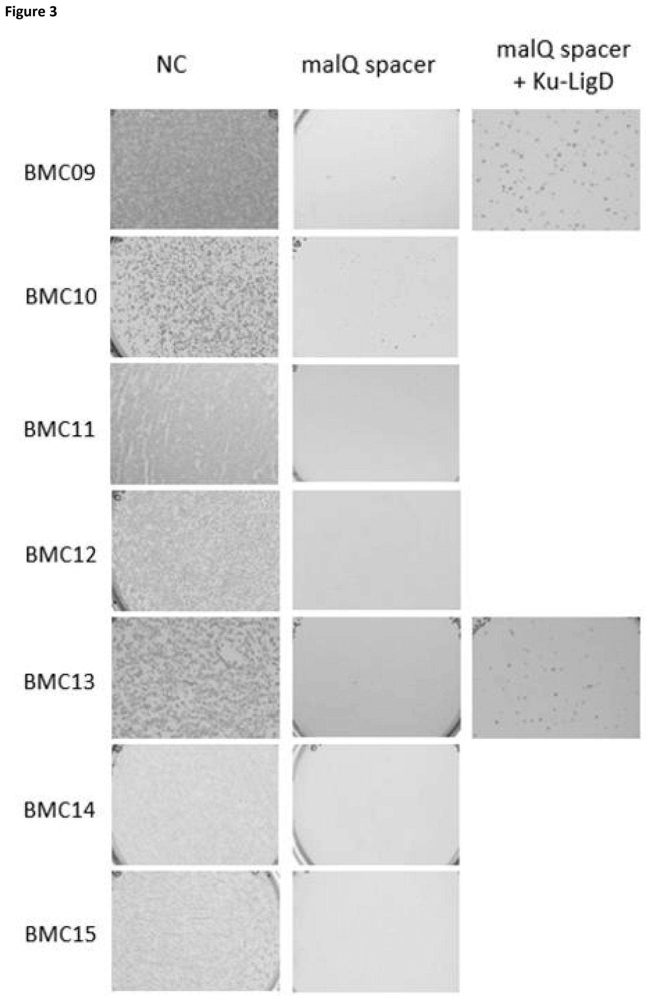

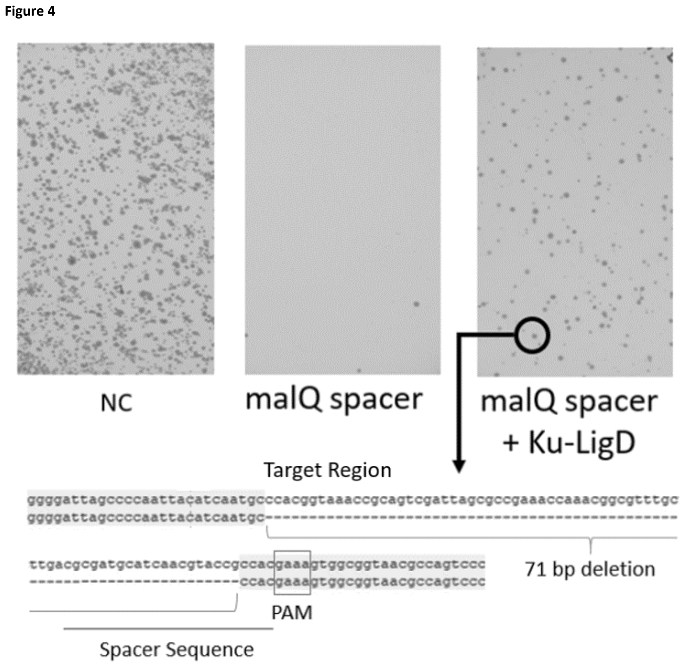

The figures show.
Fig. 1: Unrooted tree of the amino acid sequences BMC01 - BMC15
Fig. 2: Exemplary culture plates showing *E. coli* colonies 48h after transformation
   (incubated at 30°C) to visualize the colony depletion after the guided DNA targeting of the malQ gene using the BMC01, BMC02 BMC03, BMC04, BMC05, BMC07 or BMC08 nucleases and the partial recovery of colony growth (for BMC01, BMC03 and BMC04) induced by the co-transformation of the Ku-LigD proteins leading to a NHEJ mediated knock-out of the malQ gene
Fig. 3: Exemplary culture plates showing *E. coli* colonies 48h after transformation
   (incubated at 30°C) to visualize the colony depletion after the guided DNA targeting of the malQ gene using the BMC09, BMC10 BMC11, BMC12, BMC13, BMC14 or BMC15 nucleases and the partial recovery of colony growth (for BMC09 and BMC13) induced by the co-transformation of the Ku-LigD proteins leading to a NHEJ mediated knock-out of the malQ gene
Fig. 4: BMC characterization pipeline
   Cell depletion and malQ gene knock-out in *E. coli* using the BMC09 nuclease and a Ku-LigD NHEJ strategy. An excerpt of the malQ gene shows the deletion of 71 bp directly related to the target region of the BMC09 nuclease.

The examples illustrate the invention.

### EXAMPLES

### Example 1 - Habitat selection and DNA preparation

An approach for the discovery of novel Cas proteins is to access metagenomics resources in terabase scale by Next Generation Sequencing of selected environmental DNA (e.g. 1 cm³ forest soil contains ~ 2.5 x 10¹⁰ bp DNA or ~ 20 million genes) and computational identification of CRISPR-Cas systems.

To this aim, metagenome habitats were selected from different locations in Germany. Aquatic, sediment and soil habitats were sampled and preprocessed before the DNA isolation. A staggered filtration process using a variety of filters of different pore sizes (0.1 - 20 µm) were used to enrich Candidata Phyla Radiation (CPR) species containing a nearly untouched space of novel sequences (Hug et al. (2016) Nature Microbiology 1, 16048).

DNA was extracted from 0.2 and 0.1 µm filters and isolated using the PowerWater DNA isolation Kit (Qiagen).

### Example 2 - Next Generation Sequencing and sequence evaluation

DNA libraries for Next Generation Sequencing were prepared using the TruSeq PCR free Library Prep Kit (Illumina) and the metagenomics libraries were sequenced using a HiSeq 2500 (Illumina) in Rapid Mode (2 x 250 Bp) generating an average output of 50 Gbp per sample.

The generated paired end metagenome sequence libraries were assembled, annotated and evaluated to identify potential CRISPR operons by the identification of CRISPR repeats. The contigs containing the identified CRISPR repeat-spacer units were used to analyze the surrounding regions for the presence of Cas proteins. Open reading frames in close proximity to the CRISPR repeats were analyzed for their patterns and sequences by comparing them to genome and protein databases to identify potential novel Cas proteins.

BLAST nucleotide searches can be performed with the BLASTN program, score = 100, wordlength = 12, to obtain nucleotide sequences homologous to a nucleotide sequence encoding a protein of the invention. BLAST protein searches can be performed with the BLASTX program, score = 50, wordlength = 3, to obtain amino acid sequences homologous to a protein or polypeptide of the invention. To obtain gapped alignments for comparison purposes, Gapped BLAST (in BLAST 2.0) can be utilized as described in Altschul et al. (1997) Nucleic Acids Res. 25:3389. Alternatively, PSI-BLAST (in BLAST 2.0) can be used to perform an iterated search that detects distant relationships between molecules. See Altschul et al. (1997) supra. When utilizing BLAST, Gapped BLAST, PSI-BLAST, the default parameters of the respective programs (e.g., BLASTN for nucleotide sequences, BLASTX for proteins) can be used. See the website at www.ncbi.nlm.nih.gov. Alignment may also be performed manually by inspection.

### Example 3: Construction of a functional genome editing system for E. coli using selected BMC sequences

### 3.1 CRISPR/BMC-Ec vector systems for genome editing in E. coli BW25113

The necessary genetic elements for inducible expression of the BMC01, BMC02 BMC03, BMC04, BMC05, BMC07, BMC08, BMC09, BMC10, BMC11, BMC12, BMC13, BMC14 or BMC15 nuclease, for the constitutive expression of the guide RNA (gRNA) transcription and for the expression of the Ku-LigD proteins were provided on 16 separate vectors (CRISPR/BMC01-Ec, CRISPR/BMC02-Ec, CRISPR/BMC03-Ec, CRISPR/BMC04-Ec, CRISPR/BMC05-Ec, CRISPR/BMC07-Ec, CRISPR/BMC08-Ec, CRISPR/BMC09-Ec, CRISPR/BMC10-Ec, CRISPR/BMC11-Ec, CRISPR/BMC12-Ec, CRISPR/BMC13-Ec, CRISPR/BMC14-Ec, CRISPR/BMC15-Ec, CRISPR/gRNA-Ec and CRISPR/Ku-LigD-Ec)

In the following, the construction of the CRISPR/BMC01-Ec, CRISPR/gRNA-Ec and the CRISPR/Ku-LigD-Ec vector systems are described. The CRISPR/BMC02-Ec, CRISPR/BMC03-Ec, CRISPR/BMC04-Ec, CRISPR/BMC05-Ec, CRISPR/BMC07-Ec, CRISPR/BMC08-Ec, CRISPR/BMC09-Ec, CRISPR/BMC10-Ec, CRISPR/BMC11-Ec, CRISPR/BMC12-Ec, CRISPR/BMC13-Ec, CRISPR/BMC14-Ec and CRISPR/BMC15-Ec vector systems were constructed in an analogous approach as the CRISPR/BMC01-Ec vector system.

### Design of the BMC01 E. coli protein expression vector

The synthetic 3888 bps BMC01 nucleotide sequence was codon optimized for expression in *E. coli BW25113,* using a bioinformatics application provided by the gene synthesis provider GeneArt (Thermo Fisher Scientific, Regensburg, Germany), SEQ ID NO: 33. For protein expression, the resulting synthetic gene was fused to the inducible araC-ParaBAD inducible promoter system (SEQ ID NO: 34) and the fdT terminator (SEQ ID NO: 35) (Otsuka & Kunisawa, Journal of Theoretical Biology 97 (1982), 415-436) The final BMC01_*E*. *coli* protein expression cassette was inserted by Gibson Assembly Cloning (NEB, Frankurt, Germany) into an *E. coli* shuttle vector, containing all necessary genetic elements for episomal propagation and selection of recombinant *E. coli* cells.

### CRISPR/BMC01-Ec vector system

The complete nucleotide sequence of the constructed CRISPR/BMC01-Ec vector system is provided as SEQ ID NO: 36.

### CRISPR/BMC02-Ec vector system

The complete nucleotide sequence of the constructed CRISPR/BMC02-Ec vector system is provided as SEQ ID NO: 57.

### CRISPR/BMC03-Ec vector system

The complete nucleotide sequence of the constructed CRISPR/BMC03-Ec vector system is provided as SEQ ID NO: 37.

### CRISPR/BMC04-Ec vector system

The complete nucleotide sequence of the constructed CRISPR/BMC04-Ec vector system is provided as SEQ ID NO: 38.

### CRISPR/BMC05-Ec vector system

The complete nucleotide sequence of the constructed CRISPR/BMC05-Ec vector system is provided as SEQ ID NO: 58.

### CRISPR/BMC07-Ec vector system

The complete nucleotide sequence of the constructed CRISPR/BMC07-Ec vector system is provided as SEQ ID NO: 59.

### CRISPR/BMC08-Ec vector system

The complete nucleotide sequence of the constructed CRISPR/BMC08-Ec vector system is provided as SEQ ID NO: 60.

### CRISPR/BMC09-Ec vector system

The complete nucleotide sequence of the constructed CRISPR/BMC09-Ec vector system is provided as SEQ ID NO: 39.

### CRISPR/BMC10-Ec vector system

The complete nucleotide sequence of the constructed CRISPR/BMC10-Ec vector system is provided as SEQ ID NO: 61.

### CRISPR/BMC11-Ec vector system

The complete nucleotide sequence of the constructed CRISPR/BMC11-Ec vector system is provided as SEQ ID NO: 62.

### CRISPR/BMC12-Ec vector system

The complete nucleotide sequence of the constructed CRISPR/BMC12-Ec vector system is provided as SEQ ID NO: 63.

### CRISPR/BMC13-Ec vector system

The complete nucleotide sequence of the constructed CRISPR/BMC13-Ec vector system is provided as SEQ ID NO: 40.

### CRISPR/BMC14-Ec vector system

The complete nucleotide sequence of the constructed CRISPR/BMC14-Ec vector system is provided as SEQ ID NO: 64.

### CRISPR/BMC15-Ec vector system

The complete nucleotide sequence of the constructed CRISPR/BMC15-Ec vector system is provided as SEQ ID NO: 65.

### Design of the guide RNA (gRNA) expression vector

The expression of the chimeric gRNA for specific malQ gene targeting by the BMC01, BMC02, BMC03, BMC04, BMC05, BMC07, BMC08, BMC09, BMC10, BMC11, BMC12, BMC13, BMC14 or BMC15 nuclease was driven by the SacB RNA polymerase II promoter from *Bacillus megaterium* (SEQ ID NO: 41) (Richhardt et al., Applied Microbiology Biotechnology 86 (2010), 1959-1965) and terminated using the transcription T1 and T2 termination region of the *E. coli* rrnB gene (SEQ ID NO: 42) (Orosz et al., European Journal of Biochemistry 201 (1991), 653-659). The chimeric gRNA was composed of a constant 19 bps BMC family Stem-Loop sequence (SEQ ID NO: 32) fused to the malQ target-specific 24 bps spacer sequence (SEQ ID NO: 43) located inside the malQ gene of the *E. coli* genome.

The final gRNA expression cassette was inserted by Gibson Assembly Cloning (NEB, Frankurt, Germany) into an *E. coli* shuttle vector, containing all necessary genetic elements for episomal propagation and selection of recombinant *E. coli* cells.

The construction of the final CRISPR/gRNA-Ec vector system was mediated by Gibson Assembly Cloning (NEB, Frankfurt, Germany).

The complete nucleotide sequence of the constructed CRISPR/gRNA-Ec vector system is provided as SEQ ID NO: 44.

### Design of the Ku-LigD expression vector

CRISPR/BMC introduced double-strand breaks in the genome of recombinant *E*. *coli* cells were repaired by co-expresssion of the bacterial proteins Ku and LigD from *Mycobacterium tuberculosis* (Della et al. Science 306 (2004), 683-685), enabling marker-free gene knockout by error-prone non-homologous end joining (NHEJ) of targeted DNA in absence of homologous repair template (Yang et al., Biotechnology Letters 43 (2021), 2273-2281). The mycobacterial two-component NHEJ repair machine was ectopically provided on a plasmid containing different genetic elements for replication initiation and marker selection in comparison to the generated episomal BMC-expression vectors. The compatibility between the used plasmids for Ku-LigD and BMC-expression led to stable coexistence in the recombinant *E. coli cells.* For this, the Ku-LigD-expression plasmid possessed a pUC-derived high copy origin of replication (SEQ ID NO: 45) and additionally a nucleotide fragment from pRO1614, (SEQ ID NO: 46), that allowed stable maintenance of pUC-based cloning vectors in the genus *Pseudomonas* (West et al., Gene 148 (1) (1994), 81-86). The *Escherichia*/*Pseudomonas* shuttle vector contained the nourseothricin acetyltransferase gene (nat1) (SEQ ID NO: 47) as a dominant marker for selection of transformed cells (Krügel et al., Gene 127 (1) (1993), 127-131) At its N-terminus, the expressed acetyltransferase protein was fused to residues 1 to 30 of the Veg family protein from Bacillus subtilis (SEQ ID NO: 48) as generated in plasmid pHN15 (Kück and Hoff, Fungal Genetics Reports 53 (2006), article 3. https://doi.org/10.4148/1941-4765.1106). The gene expression of the fusion protein (SEQ ID NO: 49) was under the control of the constitutive veg Promoter from *Bacillus subtilis* (SEQ ID NO: 50). Both, the 822 bps nucleotide sequence of the ku gene (SEQ ID NO: 51) and the 2280 bps nucleotide sequence of ligD (SEQ ID NO: 52) from *Mycobacterium tuberculosis* were provided as synthetic DNA fragments by GeneArt (Thermo Fisher Scientific, Regensburg, Germany). The transcription and translation of both protein coding sequences were independently controlled by use of two different constitutive promoters. Genexpression of ligD was regulated by the Veg Promotor RNA polymerase II promoter from *Bacilllus subtilis* (SEQ ID NO: 53). The T7 terminator sequence (SEQ ID NO: 54) was used for transcription termination. Gene expression of ku was under the control of the SacB RNA polymerase II promoter from *Bacillus megaterium.* (SEQ ID NO: 55). For transcription termination the fdT terminator (SEQ ID NO: 35) (Otsuka & Kunisawa, Journal of Theoretical Biology 97 (1982), 415-436) was fused downstream to the coding sequence.

The complete nucleotide sequence of the constructed CRISPR/Ku-LigD-Ec vector system is provided as SEQ ID NO: 56.

The identity of all cloned DNA elements was confirmed by Sanger-Sequencing at LGC Genomics (Berlin, Germany).

### 3.2 E. coli cultivation and transformation

### Transformation of competent E. coli BW25113 cells

In brief, a single colony of recombinant E. coli BW25113 host cells harboring the (CRISPR/BMC(NN)-Ec (Example 4) expression plasmid or carrying the CRISPR/BMC(NN)-Ec and CRISPR/Ku-LigD-Ec (Example 5)) plasmids for coupled expression was inoculated into 5 ml LB-Kanamycin (25 µg/ml) medium (Example 4) or LB-Kanamycin (25 µg/ml)/Nourseothricin (50 µg/ml) medium (Example5) and incubated for 12 to 14 h at 37°C on a horizontal shaker at 250 rpm. Overnight grown pre-cultures were diluted into fresh 60 ml LB-Kanamycin (25 µg/ml) (Example 4) or LB-Kanamycin (25 µg/ml)/Nourseothricin (50 µg/ml) medium (Example 5) to obtain an optical density at 600 nm (OD600) of 0.06. The inoculated medium was incubated at 30°C on a horizontal shaker at 250 rpm until the culture reached an optical density at OD600 of 0.2. For induced BMC(NN) nuclease expression, 600 µl 20 % L-(+)-arabinose (w/v) was added to the culture at a final concentration of 0.2 % (v/v). The cultivation was continued until the culture reached an optical density at OD600 of 0.5. Subsequently 50 ml culture was transferred into one 50 ml conical tube and harvested by centrifugation at 4°C for 5 min and 4000 x g. Pelleted cells were resuspended in 50 ml ultrapure water (ice-cold) and centrifuged at 4°C for 5 min and 4000 x g.

This washing procedure was repeated twice. Washed cells were resuspended in 25 ml 10 % (w/w) glycerin and re-centrifuged at 4°C for 5 min and 4000 x g. The pelleted cells were resuspended in 5 ml 10 % (w/w) glycerin. After the final centrifugation step at 4°C for 5 min and 4000 x g, the cells were resuspended in 125 µl 10 % (w/w) glycerin. Aliquots of 25 µl competent cells were stored at - 80°C until use. For the transformation procedure, aliquots of competent cells were thawed on ice and 50 - 100 ng plasmid DNA (CRISPR/gRNA-Ec) was added. Prepared cells were electroporated at 1800 V, 25 µF and 200 Ω in *1 mm gap* size electroporation cuvettes, using the Bio-Rad Gene Pulser Xcell Electroporation System. Subsequently, 975 µL of NEB^{®} 10-beta/Stable Outgrowth Medium was added immediately after the pulse to the transformed cells. Regeneration was done at 30°C on a horizontal shaker at 250 rpm. Finally, 25 - 100 µl of the cell suspension was plated onto selective M9-Glucose agar plates supplemented with Kanamycin (25 µg/ml)/Ampicillin (100 µg/ml) (Example 4) or Kanamycin (25 µg/ml)/Ampicillin (100 µg/ml)/Nourseothrin (50 µg/ml) (Example 5). Growth of transformed *E. coli* cells on selective agar plates was screened for 48 h at 30°C.

### Example 4: E. coli depletion assay to demonstrate the DNA targeting activity of the novel BMC nucleases.

To evaluate and visualize the DNA targeting activity of the BMC family nucleases a so-called depletion assays was carried out wherein the survival rate of the *E. coli* cells after the nuclease targeting is monitored in comparison to a negative control. A lower survival rate means better nuclease activity as *E. coli* cells are not able to repair DNA double strand breaks using non homologues end joining (NHEJ) the targeting of the genomic DNA using a CRISPR nuclease leads to cell death.

For this experimental approach, the CRISPR/BMC01-Ec, CRISPR/BMC02-Ec, CRISPR/BMC03-Ec, CRISPR/BMC04-Ec, CRISPR/BMC05-Ec, CRISPR/BMC07-Ec, CRISPR/BMC08-Ec, CRISPR/BMC09-Ec, CRISPR/BMC10-Ec, CRISPR/BMC11-Ec, CRISPR/BMC12-Ec, CRISPR/BMC13-Ec, CRISPR/BMC14-Ec, or CRISPR/BMC15-Ec vector system was co-transformed together with the CRISPR/gRNA-Ec vector system containing a spacer sequence to target the *E. coli* malQ gene MalQ is coding for 4-α-glucanotransferase and is an essential gene for starch metabolism, yet not essential for survival of *E. coli* cells when cultivated on a medium containing glucose as a carbon source.

In parallel, negative control experiments using the CRISPR/BMC01-Ec, CRISPR/BMC02-Ec, CRISPR/BMC03-Ec, CRISPR/BMC04-Ec, CRISPR/BMC05-Ec, CRISPR/BMC07-Ec, CRISPR/BMC08-Ec, CRISPR/BMC09-Ec, CRISPR/BMC10-Ec, CRISPR/BMC11-Ec, CRISPR/BMC12-Ec, CRISPR/BMC13-Ec, CRISPR/BMC14-Ec, or CRISPR/BMC15-Ec vector system co-transformed together with the CRISPR/gRNA-Ec vector lacking a spacer sequence targeting the *E. coli* genome were performed to demonstrate the dependency of the Cas proteins to be guided to the target DNA region by a specific spacer.

After transformation and 48 h incubation at 30°C the culture plates were analyzed by counting the number of grown colonies.

### Results

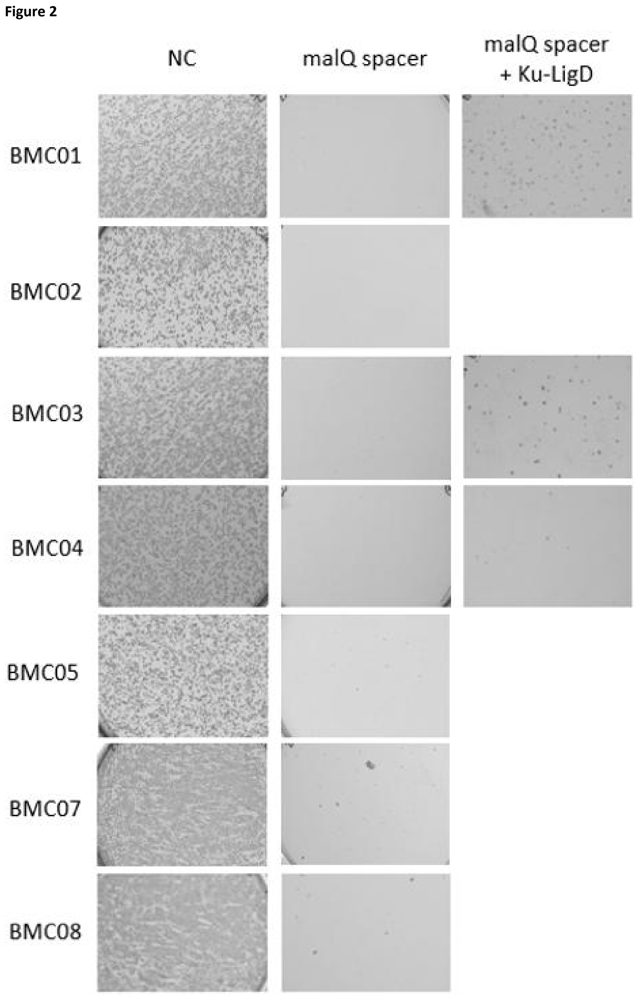

All experiments were carried out in 5 biological replicates and the results obtained from these replicates were combined to evaluate the DNA targeting of the BMC01, BMC02, BMC03, BMC04, BMC05, BMC07, BMC08, BMC09, BMC10, BMC11, BMC12, BMC13, BMC14, and BMC15 nucleases (Exemplary plates are shown in Figure 2 and Figure 3).

As described above, each of the BMC01, BMC02, BMC03, BMC04, BMC05, BMC07, BMC08, BMC09, BMC10, BMC11, BMC12, BMC13, BMC14, or BMC15 nucleases was co-transformed into *E. coli* with a gRNA targeting the *E. coli* malQ gene to visualize a) the activity and b) the DNA targeting efficiency of the BMC nucleases. After co-transformation and incubation of the plates at 30°C for 48 h, all BMC nucleases (BMC01, BMC02, BMC03, BMC04, BMC05, BMC07, BMC08, BMC09, BMC10, BMC11, BMC12, BMC13, BMC14, and BMC15) showed a strong colony reduction (>99.9%) in comparison to the negative control proving the highly efficient gRNA dependent DNA targeting of the BMC nucleases.

### Example 5: NHEJ mediated genome editing in E. coli using selected BMC nucleases and a Ku-LigD mediated strategy

To further evaluate and visualize the genome editing activity of the BMC nucleases, five representative nucleases (BMC01, BMC03, BMC04, BMC09 and BMC13) were selected and a Ku-LigD mediated strategy was used to knock-out the malQ gene located on the *E. coli* genome. As *E. coli* cells are natively not able to repair DNA double strand breaks using nonhomologous end joining (NHEJ), the targeting of the genomic DNA using a CRISPR nuclease leads to cell death.

To prevent cell death and to monitor NHEJ derived knock-out of the malQ gene (malQ is a non-essential gene and a knockout of malQ does not have any effect on the phenotype of the *E. coli* cells when cultured under the conditions used in this experimental approach), the proteins Ku-LigD (both play an important role in the DNA repair machinery) were co-transformed into the *E. coli* cells providing the cells with the ability to repair DNA double strand breaks using NHEJ (see e.g. WO 2017/109167).

For this experimental approach, the CRISPR/BMC01-Ec, CRISPR/BMC03-Ec, CRISPR/BMC04-Ec, CRISPR/BMC09-Ec or CRISPR/BMC13-Ec vector, respectively, were co-transformed together with the CRISPR/Ku-LigD-Ec vector system and the CRISPR/gRNA-Ec vector system containing a spacer sequence to target the *E. coli* malQ gene.

In parallel, negative control experiments using the CRISPR/BMC01-Ec, CRISPR/BMC03-Ec, CRISPR/BMC04-Ec, CRISPR/BMC09-Ec or CRISPR/BMC13-Ec vectors, respectively, were co-transformed together with the CRISPR/Ku-LigD-Ec vector system and the CRISPR/gRNA-Ec vector system lacking a spacer sequence targeting the *E. coli* genome were performed to demonstrate the dependency of the Cas proteins to be guided to the target DNA region by a specific spacer.

After transformation and 48 h incubation at 30°C the culture plates were analyzed by counting the number of grown colonies.

### Results

All experiments were carried out in 5 biological replicates and the results obtained from these replicates were combined to evaluate the genome editing activity of the BMC01, BMC03, BMC04, BMC09 and BMC13 nucleases. Exemplary plates are shown in Figure 2 and Figure 3.

To visualize the genome editing activity of the BMC nucleases experiments were carried out using a Ku-LigD mediated NHEJ strategy described above. First, 48h after co-transformation of the respective BMC nuclease, the gRNA (targeting the malQ gene) and the Ku-LigD expression system the plates were evaluated based on the grown colonies in comparison to the negative control and the results obtained in the cell depletion assay (Example 4). Overall, all tested BMC nucleases showed a strong colony reduction (≈98%) in comparison of the negative control but a weaker colony reduction in comparison to the results obtained in Example 4 (>99.9%). These results can be explained by the fact, that the expression of the Ku-LigD proteins enable *E. coli* to repair DNA double strand breaks using the NHEJ DNA repair mechanism. Despite from the repair of the DNA double strand break, it is known that NHEJ mediated DNA repair can be faulty, leading to indels (insertions / deletions) and frame shift mutations at the targeted position inside genome. Those cells, where the BMC mediated DNA double strand break was repaired by the NHEJ mechanism but the genomic sequence was altered due to introduced indels were able to survive the BMC treatment, as the PAM and/or spacer sequences were altered due to the indel mutation and the BMC nucleases are not able to target the genome again.

To proof the knock-out of the malQ gene, two colonies from every plate (10 colonies for every BMC nuclease) were isolated and the genomic locus of interest (inside of the malQ gene) was sequenced using Sanger sequencing.

The results of this experimental approach showed that a NHEJ mediated knock-out of the malQ gene was detectable on the sequence level for all selected BMC nucleases

BMC01: 9 out of 10 evaluated colonies showed a NHEJ mediated knock-out of the malQ gene BMC03: 7 out of 10 evaluated colonies showed a NHEJ mediated knock-out of the malQ gene BMC04: 7 out of 10 evaluated colonies showed a NHEJ mediated knock-out of the malQ gene BMC09: 10 out of 10 evaluated colonies showed a NHEJ mediated knock-out of the malQ gene BMC13: 8 out of 10 evaluated colonies showed a NHEJ mediated knock-out of the malQ gene

To demonstrate the complete workflow for the characterization of the BMC nucleases Figure 4 shows the characterization of the BMC09 nuclease: Negative control, cell depletion assay, colony growth with co-expression of Ku-LigD and the sequencing of the targeted locus (71 bp deletion inside of the malQ gene) of one *E. coli* colony.

### Summary

Taken together, the results obtained in Examples 4 and 5 demonstrate that all BMC proteins disclosed here are novel CRISPR nucleases with a high DNA targeting and genome editing efficiency and with low similarity to hitherto described CRISPR nucleases.

The subject-matter of the following items is further encompassed by the present invention.
1. A nucleic acid molecule encoding an RNA-guided DNA endonuclease, which is
   (a) a nucleic acid molecule encoding the RNA-guided DNA endonuclease comprising or consisting of the amino acid sequence of any one of SEQ ID NOs 9, 1 to 5, 7, 8 and 10 to 15;
   (b) a nucleic acid molecule comprising or consisting of the nucleotide sequence of any one of SEQ ID NOs 24, 16 to 20, 22, 23 and 25 to 30;
   (c) a nucleic acid molecule encoding a RNA-guided DNA endonuclease the amino acid sequence of which is at least 70% identical to the amino acid sequence of (a), preferably at least 80% identical, more preferably at least 90% identical, and most preferred at least 95% identical;
   (d) a nucleic acid molecule comprising or consisting of a nucleotide sequence which is at least 70% identical to the nucleotide sequence of (b), preferably at least 80% identical, more preferably at least 90% identical, and most preferred at least 95% identical;
   (e) a nucleic acid molecule which is degenerate with respect to the nucleic acid molecule of (d); or
   (f) a nucleic acid molecule corresponding to the nucleic acid molecule of any one of (a) to (d) wherein T is replaced by U.
2. The nucleic acid molecule of item 1, wherein the nucleic acid molecule is operably linked to a promoter that is native or heterologous to the nucleic acid molecule.
3. The nucleic acid molecule of item 1 or 2, wherein said nucleic acid molecule is codon-optimized for expression in a eukaryotic cell, preferably a plant cell or an animal cell.
4. A vector encoding the nucleic acid molecule of any one of items 1 to 3.
5. A host cell comprising the nucleic acid molecule of any one of items 1 to 3 or being transformed, transduced or transfected with the vector of item 4.
6. The host cell of item 5, wherein the host cell is a eukaryotic cell or a prokaryotic cell and is preferably a plant cell or an animal cell.
7. A plant, seed or a part of a plant, said part of a plant no being a single plant cell, or an animal comprising the nucleic acid molecule of any one of items 1 to 3 or being transformed, transduced or transfected with the vector of item 4.
8. A method of producing an RNA-guided DNA endonuclease comprising culturing the host cell of item 5 or 6 and isolating the RNA-guided DNA endonuclease produced.
9. An RNA-guided DNA endonuclease encoded by the nucleic acid molecule of any one items 1 to 3.
10. A composition comprising the nucleic acid molecule of any one of items 1 to 3, the vector of item 4, the host cell of item 5 or 6, the plant, seed, part of a cell or animal of item 7, the RNA-guided DNA endonuclease of item 9 or a combination thereof.
11. The composition of item 10, wherein the composition is a pharmaceutical composition or a diagnostic composition.
12. The nucleic acid molecule of any one of items 1 to 3, the vector of item 4, the host cell of item 5 or 6, the plant, seed, part of a cell or animal of item 7, the RNA-guided DNA endonuclease of item 9 or a combination thereof for use in the treatment of a disease in a subject or a plant by modifying a nucleotide sequence at a target site in the genome of the subject or plant.
13. A method of modifying a nucleotide sequence at a target site in the genome of a cell comprising introducing into said cell
   (i) a DNA-targeting RNA or a DNA polynucleotide encoding a DNA-targeting RNA, wherein the DNA-targeting RNA comprises:
      (a) a first segment comprising a nucleotide sequence that is complementary to a sequence in the target DNA; and
      (b) a second segment that interacts with the RNA-guided DNA endonuclease of item 9; and
   (ii) the RNA-guided DNA endonuclease of item 9, or the nucleic acid molecule encoding an RNA-guided DNA endonuclease of any one of items 1 to 3, or the vector of item 4, wherein the RNA-guided DNA endonuclease comprises:
      (a) an RNA-binding portion that interacts with the DNA-targeting RNA; and
      (b) an activity portion that exhibits site-directed enzymatic activity.
14. The method of item 13, wherein the cell is not the natural host of a gene encoding said RNA-guided DNA endonuclease.
15. The method of item 13 or 14, wherein in case the RNA-guided DNA endonuclease and the DNA-targeting RNA are directly introduced into the cell they are introduced in the form of a ribonucleoprotein complex (RNP).

## Claims

1. A nucleic acid molecule encoding an RNA-guided DNA endonuclease, which is
(a) a nucleic acid molecule encoding the RNA-guided DNA endonuclease comprising or consisting of the amino acid sequence of SEQ ID NO: 1;
(b) a nucleic acid molecule comprising or consisting of the nucleotide sequence of SEQ ID NO: 16;
(c) a nucleic acid molecule encoding a RNA-guided DNA endonuclease the amino acid sequence of which is at least 90% identical to the amino acid sequence of (a), preferably at least 95% identical;
(d) a nucleic acid molecule comprising or consisting of a nucleotide sequence which is at least 90% identical to the nucleotide sequence of (b), preferably at least 95% identical;
(e) a nucleic acid molecule which is degenerate with respect to the nucleic acid molecule of (d); or
(f) a nucleic acid molecule corresponding to the nucleic acid molecule of any one of (a) to (d) wherein T is replaced by U.

2. The nucleic acid molecule of claim 1, wherein the nucleic acid molecule is operably linked to a promoter that is heterologous to the nucleic acid molecule.

3. The nucleic acid molecule of claim 1 or 2, wherein said nucleic acid molecule is codon-optimized for expression in a eukaryotic cell, preferably a plant cell or an animal cell.

4. A vector encoding the nucleic acid molecule of any one of claims 1 to 3.

5. A host cell comprising the nucleic acid molecule of any one of claims 1 to 3 or being transformed, transduced or transfected with the vector of claim 4.

6. The host cell of claim 5, wherein the host cell is a eukaryotic cell or a prokaryotic cell and is preferably a plant cell or an animal cell.

7. A plant, seed or a part of a plant, said part of a plant not being a single plant cell, or an animal comprising the nucleic acid molecule of any one of claims 1 to 3 or being transformed, transduced or transfected with the vector of claim 4.

8. A method of producing an RNA-guided DNA endonuclease comprising culturing the host cell of claim 5 or 6 and isolating the RNA-guided DNA endonuclease produced.

9. An RNA-guided DNA endonuclease encoded by the nucleic acid molecule of any one of claims 1 to 3.

10. A composition comprising the nucleic acid molecule of any one of claims 1 to 3, the vector of claim 4, the host cell of claim 5 or 6, the plant, seed, part of a plant or animal of claim 7, the RNA-guided DNA endonuclease of claim 9 or a combination thereof.

11. The composition of claim 10, wherein the composition is a pharmaceutical composition or a diagnostic composition.

12. The nucleic acid molecule of any one of claims 1 to 3, the vector of claim 4, the host cell of claim 5 or 6, the plant, seed, part of a plant or animal of claim 7, the RNA-guided DNA endonuclease of claim 9 or a combination thereof for use in the treatment of a disease in a subject or a plant by modifying a nucleotide sequence at a target site in the genome of the subject or plant.

13. A method of modifying a nucleotide sequence at a target site in the genome of a cell comprising introducing into said cell
(i) a DNA-targeting RNA or a DNA polynucleotide encoding a DNA-targeting RNA, wherein the DNA-targeting RNA comprises:
(a) a first segment comprising a nucleotide sequence that is complementary to a sequence in the target DNA; and
(b) a second segment that interacts with the RNA-guided DNA endonuclease of claim 9; and
(ii) the RNA-guided DNA endonuclease of claim 9, or the nucleic acid molecule encoding an RNA-guided DNA endonuclease of any one of claims 1 to 3, or the vector of claim 4, wherein the RNA-guided DNA endonuclease comprises:
(a) an RNA-binding portion that interacts with the DNA-targeting RNA; and
(b) an activity portion that exhibits site-directed enzymatic activity,
wherein the method is not a method for the treatment of the human or animal body by therapy.

14. The method of claim 13, wherein the cell is not the natural host of a gene encoding said RNA-guided DNA endonuclease.

15. The method of claim 13 or 14, wherein in case the RNA-guided DNA endonuclease and the DNA-targeting RNA are directly introduced into the cell they are introduced in the form of a ribonucleoprotein complex (RNP).
